# EUROPEAN PATENT APPLICATION

(11) **EP 1 995 320 A2**
(43) Date of publication of application: **26.11.2008**
(21) Application number: 08156812.3
(22) Date of filing: 23.05.2008
(51) Int. Cl.: C12N 15/82

(54) **Polynucleotide markers**

(30) Priority: 23.05.2007 EP 07108777
(71) Applicant: Syngenta Participations AG, 4058 Basel (CH)
(72) Inventor: Gielen, Johannes, Jacobus, Ludgerus, 31620 Bouloc (FR); Kraft, Thomas, 227 33 Lund (SE); Pin, Pierre, 217 41 Malmö (SE)
(74) Representative: Vossius & Partner

(57) **Abstract**

The invention relates to polynucleotides that are closely linked to the bolting gene or *B* gene within the sugar beet genome and can be used for the development of molecular markers. The invention further relates to molecular markers and kits comprising said markers that can be used for mapping, identification and isolation of the bolting gene or *B* gene in the sugar beet genome and to discriminate between the annual and biennial genotype or between different haplotypes within plant groupings of sugar beet plants exhibiting a biennial genotype.

The invention also relates to assays and methods of breeding sugar beet plants involving said markers.

SEQUENCE LISTING

<110>  Syngenta Participations AG

<120>  Polynucleotide markers

<130>  N1453 EP/A BS

<150>  07 10 8777.9
<151>  2007-05-23

<160>  57

<170>  PatentIn version 3.4

<210>  1
<211>  477
<212>  DNA
<213>  Beta vulgaris

<400>  1
acgacaataa tgataaatcg ccatttggtg atacgactct taccaaggtt tttgtaggag      60

gattagcatg ggaaacacct aaacaagcct tgtttgagca ttttgaaaaa tatggtgaga     120

tctttgaggc tgttatcatc tctgataaga tcactggcag atccaagggt tatggctttg     180

tgaccttcaa ggagccagag gcagcaaaga aggcatgtga ggatgcaaca ccaatcatca     240

acgagaggcg agcaaactgc aacctagcag cgctcggggc tcgccgccca cctcgatcat     300

cgtcgacaac tccgcaaccc catcatcaac gtcaagcatc caatggaagc aatggcagcg     360

gaggtagtgg tggcaatgca ttaagcccca cctcagctgc tgcgccacct gctggtcatg     420

tgcagtggta ttatccagct gctgttgctt ctggtcgtgc tacttcaccc accccac        477


<210>  2
<211>  20
<212>  DNA
<213>  Artificial sequence

<220>
<221>  source
<223>  /note="Description of artificial sequence:
       depicts the nucleotide sequence of forward primer 9_27-F"

<400>  2
gccatttggt gatacgactc                                                  20


<210>  3
<211>  19
<212>  DNA
<213>  Artificial sequence

<220>
<221>  source
<223>  /note="Description of artificial sequence:
       depicts the nucleotide sequence of reverse primer 9_27-R"

<400>  3
agcaaactgc aacctagca                                                   19


<210>  4
<211>  860

<212>  DNA
<213>  Beta vulgaris

<400>  4
gccatttggt gatacgactc ttaccaaggt ttttgtagga ggattagcat gggaaacacc      60

taaacaagcc ttgtttgagc attttgaaaa atatggtgag atctttgagg ctgttatcat     120

ctctgataag atcactggca gatccaaggg ttatggcttt gtaagttgtc tttcacctcc     180

acattataca ctttcgcata ggggttttag tttatgtaat ccatgtcaat aaaaaaattt     240

agaaattgca tagtataggg ttgatcccaa gacctcgagt acatatatta atgttttaac     300

caccacgcta agttgtatta gtgttattat cataaagtat tatattaaaa aattaatttt     360

tttcttaccg gcgggagcat agcccgggcc tactaactcg ttttcgtcct atattgtggg     420

ctttataatc ctatcgtgtt gtatctcttt tacatctgat cttcttacaa tgccaaaaca     480

cacattgtac ctatacaaac ggtatagaaa taatgacgac tcttatagaa aatactgaca     540

ctcatcataa taaaaataag gattacacta tatttaagta gtccactatt acaattcaca     600

aactctgtta aattgatttc atctaactta gttggtagtc ttgtagagat ggtatccaag     660

gatctaggat tgaatcacat ctacatcact gctttcagcg tgtagttcat atgtgattta     720

tagttgattt gcaataataa ttaacttctt tattaaaaaa aaatgtgcag gtgaccttca     780

aggagccaga ggcagcaaag aaggcatgtg aggatgcaac accaatcatc aacgagaggc     840

gagcaaactg caacctagca                                                 860

<210>  5
<211>  610
<212>  DNA
<213>  Beta vulgaris

<400>  5
gtaagttgtc tttcacctcc acattataca ctttcgcata ggggttttag tttatgtaat      60

ccatgtcaat aaaaaaattt agaaattgca tagtataggg ttgatcccaa gacctcgagt     120

acatatatta atgttttaac caccacgcta agttgtatta gtgttattat cataaagtat     180

tatattaaaa aattaatttt tttcttaccg gcgggagcat agcccgggcc tactaactcg     240

ttttcgtcct atattgtggg ctttataatc ctatcgtgtt gtatctcttt tacatctgat     300

cttcttacaa tgccaaaaca cacattgtac ctatacaaac ggtatagaaa taatgacgac     360

tcttatagaa aatactgaca ctcatcataa taaaaataag gattacacta tatttaagta     420

gtccactatt acaattcaca aactctgtta aattgatttc atctaactta gttggtagtc     480

ttgtagagat ggtatccaag gatctaggat tgaatcacat ctacatcact gctttcagcg     540

tgtagttcat atgtgattta tagttgattt gcaataataa ttaacttctt tattaaaaaa     600

aaatgtgcag                                                            610

<210>  6
<211>  129
<212>  PRT
<213>  Artificial sequence

<220>
<221>  source
<223>  /note="Description of artificial sequence:
      depicts the putative protein sequence from the 5' coding
      region of the 9_27 gene"

<400>  6

Leu Phe Glu His Phe Glu Lys Tyr Gly Glu Ile Phe Glu Ala Val Ile
1               5                   10                  15

Ile Ser Asp Lys Ile Thr Gly Arg Ser Lys Gly Tyr Gly Phe Val Thr
            20                  25                  30

Phe Lys Glu Pro Glu Ala Ala Lys Lys Ala Cys Glu Asp Ala Thr Pro
        35                  40                  45

Ile Ile Asn Glu Arg Arg Ala Asn Cys Asn Leu Ala Ala Leu Gly Ala
    50                  55                  60

Arg Arg Pro Pro Arg Ser Ser Ser Thr Thr Pro Gln Pro His His Gln
65                  70                  75                  80

Arg Gln Ala Ser Asn Gly Ser Asn Gly Ser Gly Gly Ser Gly Gly Asn
                85                  90                  95

Ala Leu Ser Pro Thr Ser Ala Ala Ala Pro Pro Ala Gly His Val Gln
            100                 105                 110

Trp Tyr Tyr Pro Ala Ala Val Ala Ser Gly Arg Ala Thr Ser Pro Thr
        115                 120                 125

Pro

<210>  7
<211>  44
<212>  PRT
<213>  Artificial sequence

<220>
<221>  source
<223>  /note="Description of artificial sequence:
      depicts the amino acid sequence of the putative
      conserved RRM domain"

<400>  7

Leu Phe Glu His Phe Glu Lys Tyr Gly Glu Ile Phe Glu Ala Val Ile
1               5                   10                  15

Ile Ser Asp Lys Ile Thr Gly Arg Ser Lys Gly Tyr Gly Phe Val Thr
            20                  25                  30

Phe Lys Glu Pro Glu Ala Ala Lys Lys Ala Cys Glu
        35                  40

<210> 8
<211> 27
<212> DNA
<213> Artificial sequence

<220>
<221> source
<223> /note="Description of artificial sequence:
depicts the nucleotide sequence of forward primer 9_27(T1)-F"

<220>
<221> variation
<222> 9
<223> /replace="g"
/replace="c"

<400> 8
ttggtagtst tgtagagatg gtatcca 27

<210> 9
<211> 19
<212> DNA
<213> Artificial sequence

<220>
<221> source
<223> /note="Description of artificial sequence:
depicts the nucleotide sequence of reverse primer 9_27(T1)-R"

<400> 9
cctctggctc cttgaaggt 19

<210> 10
<211> 20
<212> DNA
<213> Artificial sequence

<220>
<221> source
<223> /note="Description of artificial sequence:
depicts the nucleotide sequence of primer 9_27(T1)-FAM"

<400> 10
atctaggatt gaatcacatc 20

<210> 11
<211> 19
<212> DNA
<213> Artificial sequence

<220>
<221> source
<223> /note="Description of artificial sequence:
depicts the nucleotide sequence of primer 9_27(T1)-VIC"

<400> 11
tctaggattg catcacatc 19

<210> 12
<211> 27
<212> DNA

<213>  Artificial sequence

<220>
<221>  source
<223>  /note="Description of artificial sequence:
       depicts the nucleotide sequence of forward primer 9_27(T2)-F"

<400>  12
tgccaaaaca cacattgtac ctataca                                          27


<210>  13
<211>  19
<212>  DNA
<213>  Artificial sequence

<220>
<221>  source
<223>  /note="Description of artificial sequence:
       depicts the nucleotide sequence of reverse primer 9_27(T2)-R"

<400>  13
tgcctctggc tccttgaag                                                   19


<210>  14
<211>  17
<212>  DNA
<213>  Artificial sequence

<220>
<221>  source
<223>  /note="Description of artificial sequence:
       depicts the nucleotide sequence of primer 9_27(T2)-FAM"

<400>  14
atctctacaa gactacc                                                     17


<210>  15
<211>  18
<212>  DNA
<213>  Artificial sequence

<220>
<221>  source
<223>  /note="Description of artificial sequence:
       depicts the nucleotide sequence of primer 9_27(T2)-VIC

<400>  15
catctctaca acactacc                                                    18


<210>  16
<211>  30
<212>  DNA
<213>  Artificial sequence

<220>
<221>  source
<223>  /note="Description of artificial sequence:
       depicts the nucleotide sequence of forward primer
       ED031700(T1)-F"

<400>  16
taaaggtggt aattttagag aattttagga                                       30

<210> 17
<211> 22
<212> DNA
<213> Artificial sequence

<220>
<221> source
<223> /note="Description of artificial sequence:
depicts the nucleotide sequence of reverse primer
ED031700(T1)-R"

<400> 17
gctcgttttg aaaaaatttg gg 22

<210> 18
<211> 17
<212> DNA
<213> Artificial sequence

<220>
<221> source
<223> /note="Description of artificial sequence:
depicts the nucleotide sequence of primerED031700(T1)-FAM"

<400> 18
ttaattcgca aacttct 17

<210> 19
<211> 18
<212> DNA
<213> Artificial sequence

<220>
<221> source
<223> /note="Description of artificial sequence:
depicts the nucleotide sequence of primer ED031700(T1)-VIC"

<400> 19
tttaattcgc atccttct 18

<210> 20
<211> 1408
<212> DNA
<213> Beta vulgaris

<400> 20
attttgggt cttaaactta catttaataa taatactttg ttatgttttg ctttgacatt 60

agtggttaaa ctatcacatt tctaaaaaaa aaaaaactct aacatttcat tttaaatgtc 120

atatggtcaa tttgtagtag ctatacattg ttaatatatt gaaatgaaat cataaacatg 180

caatgtctat taaaagcttg atatgagtgt catgtgacac aatacctttt agacctgagc 240

atgggtctgg tggggtcaga ccgggactat tcacgggctc cgcccgctgg cttgggacaa 300

tggcgagacg ggcttgaaat ttctagaatt ttgkccatgg cccggcccga gcccacgggc 360

ttgcrggggc ggtctcgtgt ctcttgcaag acagacccgt gtttkatgtt gttttttttt 420

gtattattaa cagttttaga ctaaaaaata aagaaggggt gtttggcccg cgagccacat 480

aggtbggctc gtgccgaacc gaggctaaaa taaaatttct tgaacgtggt aggcccacga 540

cccacataac taacgtctag tcgggcgtca agtgggcttg tgcagcgccc caacccatac 600

ccacctttag gtcctttaat aaaaataact tttaatatac ttctatagat gtagattgag 660
ttaaaattct tattttaaat acctacaaaa atttccaaat aaatgataaa catgtcatat 720
tttgaccatc actagattca ctttcctctt ttatctagag aactaatgca cagatcataa 780
aggaattatc ttgtacctag agtacttagc ttaagggatg ggagtgagct ccttaattta 840
agactcaatt tttcttactt cattttaata tttataaaaa gttgttccaa ggctatttac 900
ttcatatatg agattacaaa tgacaacttt taggcttgtt tatcaatcag ctgatagcgg 960
ttagctgtta gatgattgta ttagattatt tgaatagctg attgtattaa tagtttcgat 1020
aaaggtgttt ggtagatagc tcatagttga ttgttaatta attgtctaaa attaccatta 1080
aggacattgg cctaatattt taggttttgg gacacctagg taaaggtggt aattttagag 1140
aattttagga cataaattta atttttcttt aattcgcaaa cttctaatat caaaaagctc 1200
ttccccaaat tttttcaaaa cgagcktttt gatcccaaaa agttatttta aacctctctt 1260
tarcaaacac ataattagag aattgattag tcaaaacatc taatatgacc taaaaactct 1320
attccgcccc aatctctcat taccaaacaa ggtcttagtc agataccaat ttggtaattt 1380
actaattatg ttagaataat ttagagtg 1408

<210> 21
<211> 20
<212> DNA
<213> Artificial sequence

<220>
<221> source
<223> /note="Description of artificial sequence:
depicts the nucleotide sequence of forward primer ED031700-F"

<400> 21
ccttttagac ctgagcatgg 20

<210> 22
<211> 19
<212> DNA
<213> Artificial sequence

<220>
<221> source
<223> /note="Description of artificial sequence:
depicts the nucleotide sequence of reverse primerED031700-R"

<400> 22
agagattggg gcggaatag 19

<210> 23
<211> 551
<212> DNA
<213> Beta vulgaris

<400> 23
   cttttggctt tgtagctcct atttctagca aaaagaaacg ccctaaatca ttcttctaaa 60
   ggctcggtct tatgcaatct tggggagcta aaatgcaagt cacaagttag gcgatcaatg 120
   gtgagggttg ggaatgagtc ttacgaggtt cttggccctc cttcgggatt cgtcgcacaa 180
   ggggcaaggc aatccattca tccaactcgc ctaatttcaa agagactcta ccaaaagtta 240
   tcgttctcaa gagtcactta aagtcacaac agggcttcca aaaccatcaa ttctaatgta 300
   gggaaggtct tccaatcaag ttatgaacac ttttagccac aaatcatgca tgcaaccaag 360
   ctatcctaaa tgctgggaaa aaaaaatatt tttgtgtttt tttttttatg caaacctaac 420
   atgcaaaagc actaaaaaga acctaaatgc aaaagggaga aaaaaatgtt gagatggaga 480
   gaacacatgc taactaactc ccccaagctt aatacaaact ccctcctcgg agtgtacaag 540
   caaagagaaa g 551

<210> 24
<211> 681
<212> DNA
<213> Beta vulgaris

<400> 24
ttgtagttgc ctcgtaaatt tgtcctcaaa tctcacggcc tcaccggtat tccgtctccc 60

ttctatattt ggcattcgac tcttgggcat agtgaatcat ccaaggcact ccatcaaagg 120

ttttgttttg ggcgatgagg tttgagaata tgcattggag atagggatta aggggatata 180

ttgttatcta gtaatcatcg ctattttatt gataatgtag atataatagg agttaatgga 240

cttttttttt ttatccacag aagtagtgga ccatgggcca atgctttata agtagagaga 300

ttataataat actaggtcat gttcataaat aacaacaata taagtaaaga gcatggcgag 360

gggggctagg atttagggtt taagagattg gaggactttg gattaaaagt tagattttat 420

ttttgaacgg atatgtggat ttgggtctag ggattagatc cagactcgtt tattaggata 480

aaaaaacaaa ttcaacccga atatgtccat agtttaaaga ttaagatcct aaaataagga 540

tttagattca catatcaggg ttgggtttaa aatccattaa catctctctt agacatgtaa 600

aatgaactcg ttgaactttg tgatagttga catacttaat tttcaaatct atatcaattt 660

atagggcatt tttgcatgag g 681

<210> 25
<211> 677
<212> DNA
<213> Beta vulgaris

<400> 25
cttattgtta taacttgatc ttagattgtt ggtatttggg tagtgaaagt ggtggttgtt 60

caatctaatt gaaccggttt gtaacaaaca tcttatgttc tctttctttg ttcttgctta 120

tctattcgaa tgctcttgaa tgcttaatca atatgtatca ctatatcatt ttaacataca 180

ctatatcata tcaatataca ctatcctaaa ctcacgtgat atggatcgac taattagtgt 240

gcaccggttc atattagttc ttttaacttt tttttcaaca gagaaatgaa gaatcctcat 300

ttttaacaga gaaatggaga tagagtgaat gaaggggaga taaagtgaag gggaaattta 360

tggcgcagaa gactacagag acttgaagac attgatacga ccaggaccga ccaggaccaa 420

acgaatattg tgtttttcaa gaaaaaagaa attaaaacaa agagagaggg aaagaattct 480

ttttagaatc aaaaaggaca aattagtatt gtgtttctgg aatttttgtc ttcaataaat 540

tttggcagaa ataggaacag taatttatgt cgttacaaga atctttaata tcatttttgt 600

gcgaattcat gtcatttaga aacttaataa aatttcattc ttttgtctaa tattatatgg 660

cattgtatta gtattct 677

<210> 26
<211> 702
<212> DNA
<213> Beta vulgaris

<220>
<221> variation
<222> 628
<223> /replace="a"
/replace="c"
/replace="g"
/replace="t"

<400> 26
agggagttga aagcatatct caaggattct ttgaaagcat gttctcgtct tggatataag 60

atcattcttc ttccttacat tgaatcatat gcttcttaaa ctcacttttt actttaatta 120

aattgaccta ttagttgttc tacgataata atgacatttt tattgttgta ggaaccattg 180

gacactgatt gcaatatgtt tgccacgtaa cgaagtctac cactttaatt ctttgcgtaa 240

agaaacactg cggtccttac tagtcagatc attgttaaat aggtagccta atcgaaactc 300

aatatatgca cacttattaa tattaaaacc tctaatctta taataatgta ttgatgtaat 360

gtgatgaaga gggttattgc atctggactt cgacaaccaa aacctcctgt ttggcattta 420

gtcgaggtaa catgagtaat tttttgcatc cttttataga aacacataat acacatctaa 480

catgtcattt attgcatcct ttggtgaatt ttagtgtcta gtacaagtag gtggacgtgc 540

gtgtgggtat tacgtcatga ggttcatgct cgatatcgtc aaccgttgca aacatgttgg 600

tgatcttcaa aatgtatgta acttatangt ttccaattgt tgttggttgc atttcgggaa 660

cgaaaaggcc atttccggcc tattttggcc ttttcgctcc ca 702

<210> 27
<211> 639
<212> DNA
<213> Beta vulgaris

<400> 27
cttgacaagt ttctttatgg gttaaagcag ctccaagagc atggtatgaa aaactttcaa 60

agttcctcct tgaaaatgag tttaaaagag gaaaattgga tcgaagcttg tttttaaaat 120

caaaagggaa acatttatta gtagttcaaa tatatgttga tgatattata ttttgtgcca 180

ctaatgaatc tttgtgtaag gactttgcta atctcatgag taaagaattc gaaattagca 240

tgataggtga actcaacttc ttcttggggt tgcaaatcaa gcaaacacaa gaaggaatct 300

acatccatca acaaaagtac ataaaggaac tgctgaaaaa gtatggtttg gaggcttcca 360

aagtaaatca tacacctatg ggaactttaa ccaaattgga tgaggatact ataggaacaa 420

gtgtggatca aaccaagtat cgaggtatga ttggctcatt attatatcaa actgcaagtc 480

gtccggatat tgcgtatagt gtaggggtta tgtgcaaggt ttcaagccaa tcaaaagaaa 540

tctcatctca cagcagtaaa gagaatccta agatacctca aaggaacaga tgatttgagc 600

ctattctatt caagaagtga tacctttgaa ccgaaagga 639

<210> 28
<211> 715
<212> DNA
<213> Beta vulgaris

<220>
<221> variation
<222> 685
<223> /replace="a"
/replace="c"
/replace="g"
/replace="t"

<400> 28
gatgattcta atataccttc ttgatcttcg atataaaata aaaaagatca agtccatgtg 60

gaacaaggat gacttccagg ggtatgcgct aatagagttt ggtatatctg atgaagatgt 120

tagatcttct atgaaattag aaagtcagtt cgaggaggaa gggcatggga aggttgcatg 180

gctttcggga aatgttgatg caagaccgta tctttgggtg gctaccatac atgatcgtca 240

attattaaga agcaatgttg attatatgac aatccccaat gaatggaggg ctttggcaaa 300

aacagaagct aagaatgaca taatggatgc agctggggcg gtaacatctt ggctagcaaa 360

caaagaccta atgacataat ggttgatatt tttttagata tcttttctgt cagctttttc 420

gtgtactttg tagtttctta tttattaata aaatatttaa agttcatgat aatattttca 480

ttgtcgttgt tgtttgttct ttatgatagt atttccatcg tcatttttttc aatgaatctg 540

taatgacatg aaattgaagg agactgacaa tggatacaaa tataatgaca taaaatataa 600

gacaaaagaa tgaaatgttt taaagtttct aaattatatg aacgcgcacc aaaatgatat 660

taaagactag taacgaatga cacanatttc ataatgctaa caaagcattg actaa 715

<210> 29
<211> 697
<212> DNA
<213> Beta vulgaris

<400> 29
cttaccatat tatcttccca aatcatcttt tttaaagact ctgacacctc tttccatgag 60

tggagaatgg agagcttatg catgcaaatc ccaacttgca tggtgtttat gccaaattct 120

gatcaggcag aacttagctg aggatctaca gctaatggag atgcagctac aaacaataaa 180

agatactagc tacgaaattg aaagagaatg caaagcaaag gcgaagacac caagaaatgg 240

tgacgcggaa aaccccaaga agagggtaaa aaccgcgggt ggaattgagt ccaccaatcc 300

actatgtagg aagattacaa cttatagcta gtagctagca gcttgtagct tatgctgaat 360

acacttgtaa atatttgtga aagtaaatgg aagtgttcta atgtaagcaa tgttaatgaa 420

ctgccctttt ccccaatact tctactccta tttatatcca tacatttatt gtaacgttgc 480

tttacatgtt tacgttgcat aactaatctc ctaatatgat gccatgatct cctaatatca 540

aggagtatct ccacttctgc ttaatgactc tgtcaaccct attttgtagg cgcaacagca 600

ttacttgact tggtcttgat cagtatccaa tgctctttga atcgaattta ttcaactccc 660

ttcgttcctg cttgctgtag ctgctacctg gtaactt 697

<210> 30
<211> 376
<212> DNA
<213> Beta vulgaris

<400> 30
ctattaaact catgaatgaa cattcaactc aataaattcc acacccaaac tctaaaaata 60

aaaaacccca attcaatttc aatctaaaaa ccctcactac ataatttcaa aagaaaactt 120

aaattagttg aggaattcat cttaaccact aatatagaag attggggatg agaaataact 180

tgcaaacacc cctaattcaa gcatcaaatg ctccaaatta cagcaacgac gacccccctt 240

ttcttccttg tgccgcctac tctatggcat catctctagt ggcggttgtg gttgtgtcgt 300

cggcacggtg gtggttgtgt gatgaatgga gaatgttaaa gattttgaga gaagaagatg 360

gtgaagatgg tgaagt 376

<210> 31
<211> 623
<212> DNA
<213> Beta vulgaris

<400> 31
cttgaagggt tgtagccttc agcagccctt ctacttagaa gggttgcgct acttcaaaaa 60

gcgcagccct tctgttaatt tttttttttt ttgtaaatta atacaacacc ctgcagcatg 120

cagcccttcc cacccacagc ctctacccac ccccaccctt tcccacccct gtccaccccc 180

ctgcccaccc ccagcccacc cctgcccacc cttgcacagc ccccagccca ccccctgcct 240

ctgcccacgc ccacacccct acccaccccc agcccaccta gacctgctac ccacccatgc 300

ccacccctgc ccacccccag cccacccctg cacaaccccc agtccaccct tctgccacac 360

cccatccaca ccccctgccc acccctctgc cgcacccctg ccaccagccc tgcacggttg 420

cacccccatc cccagcccac ccagcagccc ccaccacccc acccccaccc cccacccaaa 480

acctcccttg gacaaaaaca aatgcaataa catatgacaa gtggagaaag gtaataatta 540

acttcaagta tatttaataa tgtaaaaagt gtacatagaa tcgatagaaa ctaatacatt 600

cgagctaaaa tgtatcatat aca 623

<210> 32
<211> 646
<212> DNA

<213>  Beta vulgaris

<400>  32
gtaaacttct tcctcaagat caccatggat aaatgcacta tcaacatcca tctaatgaag      60

ttcccaattc ttagcagcag caatacctag aaaagtacga accgtcacca tgttggcaac     120

aggggcaaag gtatctgtgt aatcaaggcc tttagtttga tgataacgaa aaacaacaag     180

aagatatttt aaccgttcaa gagtcccgtc aaaattgaat ttgttcctat agacccattg     240

gttacccaaa gcacgttttc ctagaggtaa gagctccatg gtccaagtcc taatttcttc     300

caaggcgcga attttattgt tcatggattc tcgccaacta gcatctctca tggcctattt     360

aaaagagcgc ggttcttgag cagtagttac cgctgcaaga aaatttcggt atggcaataa     420

aaaactgtca caagttatat aatgtgctat aggataggaa gtacctgagg gatgagaaga     480

agcatgagaa ggggaggatg gactcttagc ttgcaccgtg tgagtaacat agtcacgcaa     540

aagaacagag ggctgcttag cccgcaaacc acgcccaaga gaaaatagct catcagaaac     600

gctgacagat ggccagtaga caaaagccca ggttcgagct tagagc                    646


<210>  33
<211>  388
<212>  DNA
<213>  Beta vulgaris

<400>  33
cttcctacta ttctaaacta actattaaac tcatgaatga acattcaact caataaattc      60

cacacccaaa ctctaaaata aaaaacccca attcaatttc aatctaaaaa ccctcactac     120

ataattttaa aagaaaactt aaattagttg aggaattcat cttacccact aatatagaag     180

attggggatg agaaataact tgcaaacgcc cctaattcga gcaccaaatg ctccaaatta     240

cagcaacgac gacccccctt ttcttccttg cgccgcctac tctatggcat cacctctagt     300

ggcggttgtg gttgtgttgt cggcacgttg gtggttgtgt gatgaatgga gaatgttaaa     360

gattttgaga gaagaagatg gtgaagat                                        388


<210>  34
<211>  20
<212>  DNA
<213>  Artificial Sequence

<220>
<221>  source
<223>  /note="Description of artificial sequence:
       depicts the nucleotide sequence of forward primer HiNK6986"

<400>  34
aggtttgggt actaccacac                                                  20


<210>  35
<211>  22
<212>  DNA
<213>  Artificial Sequence

<220>
<221>  source

<223>  /note="Description of artificial sequence:
       depicts the nucleotide sequence of forward primer HiNK6989"

<400>  35
tgatgtccaa tgttacgtta cg                                              22


<210>  36
<211>  20
<212>  DNA
<213>  Artificial Sequence

<220>
<221>  source
<223>  /note="Description of artificial sequence:
       depicts the nucleotide sequence of forward primer HiNK7534"

<400>  36
atgcaagacc gtatctttgg                                                 20


<210>  37
<211>  22
<212>  DNA
<213>  Artificial Sequence

<220>
<221>  source
<223>  /note="Description of artificial sequence:
       depicts the nucleotide sequence of forward primer HiNK7536"

<400>  37
gtatccattg tcagtctcct tc                                              22


<210>  38
<211>  20
<212>  DNA
<213>  Artificial Sequence

<220>
<221>  source
<223>  /note="Description of artificial sequence:
       depicts the nucleotide sequence of forward primer HiNK7538"

<400>  38
agactctgac acctctttcc                                                 20


<210>  39
<211>  19
<212>  DNA
<213>  Artificial Sequence

<220>
<221>  source
<223>  /note="Description of artificial sequence:
       depicts the nucleotide sequence of forward primer HiNK7539"

<400>  39
agcataagct acaagctgc                                                  19


<210>  40
<211>  19
<212>  DNA
<213>  Artificial Sequence

<220>
<221> source
<223> /note="Description of artificial sequence:
depicts the nucleotide sequence of forward primer HiNK7563"

<400> 40
tgtttgccac gtaacgaag 19

<210> 41
<211> 19
<212> DNA
<213> Artificial Sequence

<220>
<221> source
<223> /note="Description of artificial sequence:
depicts the nucleotide sequence of forward primer HiNK7565"

<400> 41
ccgaaatgca accaacaac 19

<210> 42
<211> 22
<212> DNA
<213> Artificial Sequence

<220>
<221> source
<223> /note="Description of artificial sequence:
depicts the nucleotide sequence of forward primer HiNK7766"

<400> 42
gtgccactaa tgaatctttg tg 22

<210> 43
<211> 22
<212> DNA
<213> Artificial Sequence

<220>
<221> source
<223> /note="Description of artificial sequence:
depicts the nucleotide sequence of forward primer HiNK7768"

<400> 43
ctctttactg ctgtgagatg ag 22

<210> 44
<211> 21
<212> DNA
<213> Artificial Sequence

<220>
<221> source
<223> /note="Description of artificial sequence:
depicts the nucleotide sequence of forward primer HiNK7543"

<400> 44
gcaaacaccc ctaattcaag c 21

<210> 45
<211> 19
<212> DNA

<213>  Artificial Sequence

<220>
<221>  source
<223>  /note="Description of artificial sequence:
      depicts the nucleotide sequence of forward primer HiNK7545"

<400>  45
tcatcacaca accaccacc                                                    19


<210>  46
<211>  21
<212>  DNA
<213>  Artificial Sequence

<220>
<221>  source
<223>  /note="Description of artificial sequence:
      depicts the nucleotide sequence of forward primer HiNK7550"

<400>  46
aagttcccaa ttcttagcag c                                                 21


<210>  47
<211>  20
<212>  DNA
<213>  Artificial Sequence

<220>
<221>  source
<223>  /note="Description of artificial sequence:
      depicts the nucleotide sequence of forward primer HiNK7552"

<400>  47
gcttcttctc atccctcagg                                                   20


<210>  48
<211>  22
<212>  DNA
<213>  Artificial Sequence

<220>
<221>  source
<223>  /note="Description of artificial sequence:
      depicts the nucleotide sequence of forward primer HiNK7554"

<400>  48
caactcaata aattccacac cc                                                22


<210>  49
<211>  19
<212>  DNA
<213>  Artificial Sequence

<220>
<221>  source
<223>  /note="Description of artificial sequence:
      depicts the nucleotide sequence of forward primer HiNK7556"

<400>  49
ctccattcat cacacaacc                                                    19


<210>  50

<211> 21
<212> DNA
<213> Artificial Sequence

<220>
<221> source
<223> /note="Description of artificial sequence:
depicts the nucleotide sequence of forward primer HiNK7522"

<400> 50
gggcatagtg aatcatccaa g 21

<210> 51
<211> 21
<212> DNA
<213> Artificial Sequence

<220>
<221> source
<223> /note="Description of artificial sequence:
depicts the nucleotide sequence of forward primer HiNK7525"

<400> 51
cccaaatcca catatccgtt c 21

<210> 52
<211> 20
<212> DNA
<213> Artificial Sequence

<220>
<221> source
<223> /note="Description of artificial sequence:
depicts the nucleotide sequence of forward primer HiNK7528"

<400> 52
agtgaaagtg gtggttgttc 20

<210> 53
<211> 19
<212> DNA
<213> Artificial Sequence

<220>
<221> source
<223> /note="Description of artificial sequence:
depicts the nucleotide sequence of forward primer HiNK7532"

<400> 53
   cgccataatt tcccccttc 19

<210> 54
<211> 22
<212> DNA
<213> Artificial Sequence

<220>
<221> source
<223> /note="Description of artificial sequence:
depicts the nucleotide sequence of forward primer HiNK6853"

<400> 54
ccatctctac aagactacca ac 22

<210> 55
<211> 790
<212> DNA
<213> Beta vulgaris

<220>
<221> variation
<222> 631
<223> /replace="a"
/replace="c"

<400> 55
aacacctaaa caagccttgt ttgagcattt tgaaaaatat ggtgagatct ttgaggctgt 60

tatcatctct gataagatca ctggcagatc caagggttat ggctttgtaa gttttctttc 120

acctccacat tatacacttt cgcatatgga gttttagttc atgtaatcca tgtcaataaa 180

aaaatttaga aattgcgtag tatagggttg atcccaagac cttgagtacg tatattaaag 240

ttttaaccac caggctaagt tgtattagtg ttattatcat aaagtattat attaaaaaat 300

taaatttttt cttatcggcc ggagcatagc ccaggcctac taactcgttt tcgtcctata 360

ttgtgggctt tataatcgta tcgtgttgta tctcttttac atctgatctt cttacaatgc 420

caaaacacac attgtaccta tacaaacggt atagaaataa tgacgactct tatagaaaat 480

actgacactc atcataataa aaataaggat tacactatat ttaagtagtc cactattaca 540

attcacaaac tctgttaaat tgatttcatc taactttcat ctaacttagt tggtagtctt 600

gtagagatgg tatccaagga tctaggattg matcacatct acatcactgc tttcagcata 660

tattcatatg tgatttatag ttgatttgca ataataatta acttgtttat taaaaaaaat 720

gtgcaggtga ccttcaagga gccagaggca gcaaagaagg catgtgagga tgcaacacca 780

atcatcaacg 790

<210> 56
<211> 790
<212> DNA
<213> Beta vulgaris

<220>
<221> variation
<222> 598
<223> /replace="g"
/replace="c"

<400> 56
aacacctaaa caagccttgt ttgagcattt tgaaaaatat ggtgagatct ttgaggctgt 60

tatcatctct gataagatca ctggcagatc caagggttat ggctttgtaa gttttctttc 120

acctccacat tatacacttt cgcatatgga gttttagttc atgtaatcca tgtcaataaa 180

aaaatttaga aattgcgtag tatagggttg atcccaagac cttgagtacg tatattaaag 240

ttttaaccac caggctaagt tgtattagtg ttattatcat aaagtattat attaaaaaat 300

taaatttttt cttatcggcc ggagcatagc ccaggcctac taactcgttt tcgtcctata 360

ttgtgggctt tataatcgta tcgtgttgta tctcttttac atctgatctt cttacaatgc 420

caaaacacac attgtaccta tacaaacggt atagaaataa tgacgactct tatagaaaat 480

actgacactc atcataataa aaataaggat tacactatat ttaagtagtc cactattaca 540

attcacaaac tctgttaaat tgatttcatc taactttcat ctaacttagt tggtagtstt 600

gtagagatgg tatccaagga tctaggattg aatcacatct acatcactgc tttcagcata 660

tattcatatg tgatttatag ttgatttgca ataataatta acttgtttat taaaaaaaat 720

gtgcaggtga ccttcaagga gccagaggca gcaaagaagg catgtgagga tgcaacacca 780

atcatcaacg 790

<210> 57
<211> 1408
<212> DNA
<213> Beta vulgaris

<220>
<221> variation
<222> 1179
<223> /replace="a"
/replace="t"

<220>
<221> variation
<222> 1180
<223> /replace="a"
/replace="c"

<400> 57
   atttttgggt cttaaactta catttaataa taatactttg ttatgttttg ctttgacatt 60
   agtggttaaa ctatcacatt tctaaaaaaa aaaaaactct aacatttcat tttaaatgtc 120
   atatggtcaa tttgtagtag ctatacattg ttaatatatt gaaatgaaat cataaacatg 180
   caatgtctat taaaagcttg atatgagtgt catgtgacac aatacctttt agacctgagc 240
   atgggtctgg tggggtcaga ccgggactat tcacgggctc cgcccgctgg cttgggacaa 300
   tggcgagacg ggcttgaaat ttctagaatt ttgtccatgg cccggcccga gcccacgggc 360
   ttgcaggggc ggtctcgtgt ctcttgcaag acagacccgt gtttgatgtt gttttttttt 420
   gtattattaa cagttttaga ctaaaaaata aagaaggggt gtttggcccg cgagccacat 480
   aggttggctc gtgccgaacc gaggctaaaa taaaatttct tgaacgtggt aggcccacga 540
   cccacataac taacgtctag tcgggcgtca agtgggcttg tgcagcgccc caacccatac 600
   ccacctttag gtcctttaat aaaaataact tttaatatac ttctatagat gtagattgag 660
   ttaaaattct tattttaaat acctacaaaa atttccaaat aaatgataaa catgtcatat 720
   tttgaccatc actagattca ctttcctctt ttatctagag aactaatgca cagatcataa 780
   aggaattatc ttgtacctag agtacttagc ttaagggatg ggagtgagct ccttaattta 840
   agactcaatt tttcttactt cattttaata tttataaaaa gttgttccaa ggctatttac 900
   ttcatatatg agattacaaa tgacaacttt taggcttgtt tatcaatcag ctgatagcgg 960
   ttagctgtta gatgattgta ttagattatt tgaatagctg attgtattaa tagtttcgat 1020
   aaaggtgttt ggtagatagc tcatagttga ttgttaatta attgtctaaa attaccatta 1080
   aggacattgg cctaatattt taggttttgg gacacctagg taaaggtggt aattttagag 1140
   aattttagga cataaattta atttttcttt aattcgcawm cttctaatat caaaaagctc 1200
   ttccccaaat tttttcaaaa cgagcgtttt gatcccaaaa agttatttta aacctctctt 1260
   tagcaaacac ataattagag aattgattag tcaaaacatc taatatgacc taaaaactct 1320
   attccgcccc aatctctcat taccaaacaa ggtcttagtc agataccaat ttggtaattt 1380
   actaattatg ttagaataat ttagagtg 1408

## Description

The present invention is in the area of marker-assisted breeding and quality control of sugar beet seed. In particular, the invention relates to polynucleotides that are closely linked to the bolting gene or *B* gene within the sugar beet genome and can be used for the development of molecular markers. The invention further relates to molecular markers and kits comprising said markers that can be used for mapping, identification and isolation of the bolting gene or B gene in the sugar beet genome and to discriminate between the annual and biennial genotype or between different haplotypes within plant groupings of sugar beet plants exhibiting a biennial genotype.

The cultivated sugar beet (Beta *vulgaris* ssp. *vulgaris* L.) is a biennial plant which forms a storage root and a leaf rosette in the first year. Shoot elongation (bolting) and flower formation starts after a period of low temperature. In contrast, many wild beets of the genus *B. vulgaris* ssp. *maritima* show an annual growing habit due to the presence of the bolting gene *B* at the B locus, which was mapped to the central region of chromosome 2. The dominant allele of locus B. is abundant in wild beets and causes bolting under long days without the cold requirement usually essential for biennial cultivars (Abe *et al.,* 1997) carrying the recessive allele.
Bolting (stem elongation) is the first step clearly visible in the transition from vegetative to reproductive growth.
In cultivated sugar beet, bolting is an undesirable phenomenon, as it results in reduction of yield and gives rise to problems during harvesting and sugar extraction. Owing to the incomplete penetrance of the *B* allele and its environmental dependence, closely linked molecular markers are needed to screen its presence in breeding lines.
Commercial seed productions for sugar beet are often done in regions, where annual weed beets are growing, which can cause pollen contamination in the seed productions, resulting in annuals in the commercial seed. This is not acceptable to the customers. To identify contaminations with annuals, commercial seed lots are grown in regions where no wild annual beets are growing directly after harvesting the seed. The plants are not vernalized and contaminations are identified by the presence of boilers. Replacing this test with a marker-based screening assay would be highly desirable, as results could be obtained earlier, which would lead to cost savings in seed processing.
A marker-based approach could also be advantageously used in sugar beet breeding, e.g. to speed up the breeding process, or to introduce new variation from wild sea beets. In these cases, it is important to have a marker tightly linked to the B gene to be able to select annuals or biennials accurately.

The present invention now provides the means to develop such markers.
In particular, the present invention relates to a polynucleotide or an informative fragment thereof, particularly an isolated polynucleotide or an informative fragment thereof, identified in the sugar beet genome including variants and derivatives thereof, which polynucleotide is genetically closely linked to the bolting gene or *B* gene. The invention further relates to the use of said polynucleotide for the development of markers that can be used for mapping, identification and isolation of the bolting gene or *B* gene in the sugar beet genome.

In one aspect of the invention, the polynucleotide according to the invention shows perfect co-segregation with the bolting gene (*B* gene) associated phenotype in sugar beet.

In one embodiment, the invention relates to a polynucleotide including an informative fragment thereof according to the invention and as described herein before, which is obtainable from a genomic DNA region that maps at a distance of 1 cM upstream of markers MP0176 and GJ01 and co-segregates with marker GJ131.

In another embodiment, the invention relates to a polynucleotide including an informative fragment thereof, particularly an isolated polynucleotide, according to the invention and as described herein before which is obtainable from a genomic DNA located in the interval delimited by markers GJ131 and GJ01.

In one embodiment, the invention relates to a polynucleotide according to the invention and as described herein before, which is obtainable from a genomic DNA region that maps at a distance of less than 1 cM, particularly of less than 0.75 cM, more particularly of less than 0.5 cM, even more particularly of less than 0.3 cM, but especially of less than 0.25 cM relative to the B gene.

In one embodiment of the invention, a polynucleotide or an informative fragment thereof, particularly an isolated polynucleotide or an informative fragment thereof, is provided which polynucleotide is obtainable from a genomic sugar beet DNA genetically linked to the bolting gene or *B* gene in the sugar beet genome and comprises one or more of the following elements:
a) an intronic region that can be obtained from the sugar beet genome by PCR amplification based on forward primer 9_27-F and reverse primer 9_27-R as given in SEQ ID NO: 2 and SEQ ID NO: 3, respectively, particularly a DNA fragment exhibiting a nucleotide sequence as depicted in SEQ ID NOs: 5, or a sequence that has at least 70%, particularly at least 75%, more particularly at least 80%, even more particularly at least 85%, but especially at least 90% and up to at least 95%-99% sequence identity therein;
b) a polynucleotide fragment comprising a nucleotide sequence which has 70%, particularly 75%, more particularly 80%, even more particularly 85%, but especially 90% and up to 95%-99% sequence identity with a nucleotide sequence depicted in SEQ ID NO: 4;
c) a polynucleotide, particularly an isolated polynucleotide, fragment which, after splicing, has 70%, particularly 75%, more particularly 80%, even more particularly 85%, but especially 90% and up to 95%-99% sequence identity with a cDNA sequence shown in SEQ ID NO:1 and, optionally, in addition
d) a nucleotide sequence encoding a RNA recognition motif domain (RRM).

In one embodiment of the invention, a polynucleotide or an informative fragment thereof, particularly an isolated polynucleotide or an informative fragment thereof, is provided which polynucleotide is obtainable from a genomic sugar beet DNA genetically linked to the bolting gene or B gene in the sugar beet genome and comprises one or more of the following elements:
a) an intronic region that can be obtained from the sugar beet genome by PCR amplification based on forward primer 9_27-F and reverse primer 9_27-R as given in SEQ ID NO: 2 and SEQ ID NO: 3, respectively, particularly a DNA fragment exhibiting a nucleotide sequence as depicted in SEQ ID NOs: 5, or a sequence that has at least 70%, particularly at least 75%, more particularly at least 80%, even more particularly at least 85%, but especially at least 90% and up to at least 95%-99% sequence identity therein;
b) a polynucleotide fragment comprising a nucleotide sequence which has 70%, particularly 75%, more particularly 80%, even more particularly 85%, but especially 90% and up to 95%-99% sequence identity with a nucleotide sequence depicted in SEQ ID NO: 4; and, optionally, in addition
c) a nucleotide sequence encoding a RNA recognition motif domain (RRM).

In one embodiment, the polynucleotide according to the invention comprises a nucleotide sequence that has at least 70%, particularly at least 75%, more particularly at least 80%, even more particularly at least 85%, but especially at least 90% and up to at least 95%-99% sequence identity with the nucleotide sequence depicted in SEQ ID NO: 1.

In one embodiment, the polynucleotide according to the invention comprises a nucleotide sequence that has at least 70%, particularly at least 75%, more particularly at least 80%, even more particularly at least 85%, but especially at least 90% and up to at least 95%-99% sequence identity with the nucleotide sequence depicted in SEQ ID NO: 4.

In one embodiment, the invention relates to a polynucleotide including an informative fragment thereof, particularly an isolated polynucleotide, comprising an intronic region that has at least 70%, particularly at least 75%, more particularly at least 80%, even more particularly at least 85%, but especially at least 90% and up to at least 95%-99% sequence identity with the nucleotide sequence depicted in SEQ ID NO: 5.

All individual numerical values, which fall into the range from between 70% - 99%, i.e. 71%, 72%, 73%, 74%, 75% ... 90%, 91%, 92%, 93%, 94%, 95%, 96%, 97%, 98%, 99% should likewise be covered by the present invention.

In a specific embodiment, the polynucleotide according to the invention comprises a nucleotide sequence that has the nucleotide sequence depicted in SEQ ID NO:1.

In a specific embodiment, the polynucleotide according to the invention comprises a nucleotide sequence that has the nucleotide sequence depicted in SEQ ID NO: 4.

In a specific embodiment, the polynucleotide according to the invention comprises a nucleotide sequence that has the nucleotide sequence depicted in SEQ ID NO: 5.

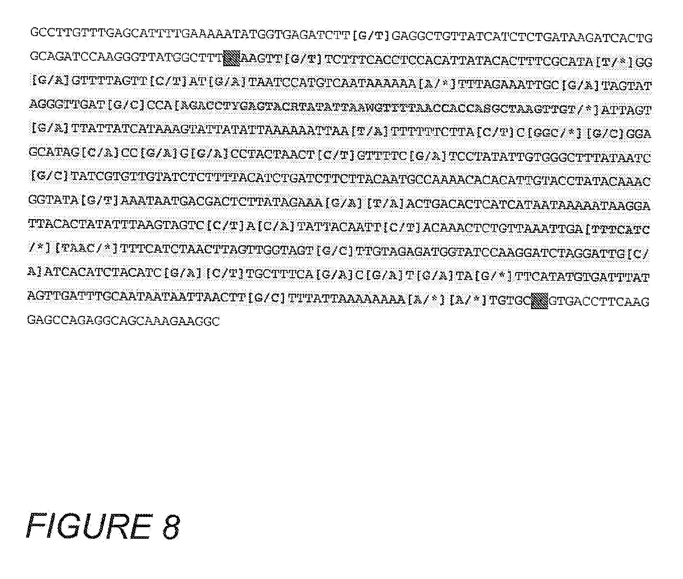

In a specific embodiment, the invention relates to a polynucleotide, particularly an isolated polynucleotide, comprising an intronic region as shown in Fig. 8.

In another specific embodiment, the invention relates to a polynucleotide or an informative fragment thereof, particularly an isolated polynucleotide or an informative fragment thereof, comprising an intronic region which is encompassed by the donor (GT) splicing site at position 161/162 and the acceptor (AG) splicing site at position 769/770 of the nucleotide sequence depicted in SEQ 1D NO: 4.

In a specific embodiment, the invention relates to a polynucleotide or an informative fragment thereof, particularly an isolated polynucleotide or an informative fragment thereof, comprising an intronic region as shown in SEQ ID NO: 5.

In a specific embodiment, the polynucleotide according to the invention comprises a RNA recognition motif domain (RRM) which has at least 70%, particularly at least 75%, more particularly at least 80%, even more particularly at least 85%, but especially at least 90% and up to at least 95% - 99% sequence identity with the RRM motif as shown in SEQ ID NO: 7.

In still another specific embodiment, the polynucleotide according to the invention comprises a nucleotide sequences as shown in SEQ ID NO: 55 comprising an A/C SNP at position #631.

In still another specific embodiment, the polynucleotide according to the invention comprises a nucleotide sequences as shown in SEQ ID NO: 56 comprising a G/C SNP at position #598.

In still another specific embodiment, the polynucleotide according to the invention comprises a nucleotide sequences as shown in SEQ ID NO: 57 comprising an A/T SNP at position #1179 and an A/C SNP at position 1180.

In one embodiment, the invention relates to a polynucleotide, particularly an isolated polynucleotide, or an informative part or fragment thereof represented by a nucleotide sequence obtainable from at least one BAC selected from a group of partially overlapping BACs, which BACs can be identified and isolated from a BAC library developed from the biennial commercial cultivar H20 and tiled based on their BAC end sequences (BES) which can be amplified using at least one primer pair selected from the group consisting of:
- primer pair 2 represented by a forward primer of SEQ ID NO: 34 and a reverse primer of SEQ ID NO: 35,
- primer pair 3 represented by a forward primer of SEQ ID NO: 21 and a reverse primer of SEQ ID NO: 22,
- primer pair 4 represented by a forward primer of SEQ ID NO: 36 and a reverse primer of SEQ ID NO: 37,
- primer pair 5 represented by a forward primer of SEQ ID NO: 38and a reverse primer of SEQ ID NO: 39,
- primer pair 6 represented by a forward primer of SEQ ID NO: 40 and a reverse primer of SEQ ID NO: 41,
- primer pair 7 represented by a forward primer of SEQ ID NO: 42 and a reverse primer of SEQ 10 NO: 43,
- primer pair 8 represented by a forward primer of SEQ ID NO: 44 and a reverse primer of SEQ ID NO: 45,
- primer pair 9 represented by a forward primer of SEQ ID NO: 46 and a reverse primer of SEQ ID NO: 47,
- primer pair 10 represented by a forward primer of SEQ ID NO: 48 and a reverse primer of SEQ ID NO: 49,
- primer pair 11 represented by a forward primer of SEQ ID NO: 50 and a reverse primer of SEQ ID NO: 51, and
- primer pair 12 represented by a forward primer of SEQ ID NO: 52 and a reverse primer of SEQ ID NO: 53.

In a specific embodiment, a polynucleotide, particularly an isolated polynucleotide, according to the invention and as described herein before is provided or an informative part or fragment thereof, which polynucleotide is characterized by at least one end sequence which can be amplified using primer pair 2 represented by a forward primer of SEQ ID NO: 34 and a reverse primer of SEQ ID NO: 35 and/or primer pair 3 represented by a forward primer of SEQ ID NO: 21 and a reverse primer of SEQ ID NO: 22.

In another specific embodiment, a polynucleotide, particularly an isolated polynucleotide, according to the invention and as described herein before is provided or an informative part or fragment thereof, which polynucleotide is characterized by at least one end sequence which can be amplified using primer pair 4 represented by a forward primer of SEQ ID NO: 36 and a reverse primer of SEQ ID NO: 37 and/or primer pair 5 represented by a forward primer of SEQ ID NO: 38 and a reverse primer of SEQ ID NO: 39.

In another specific embodiment, a polynucleotide, particularly an isolated polynucleotide, according to the invention and as described herein before is provided or an informative part or fragment thereof, which polynucleotide is characterized by the end sequence of SEQ ID NO: 20.

In another specific embodiment, a polynucleotide, particularly an isolated polynucleotide, according to the invention and as described herein before is provided or an informative part or fragment thereof, which polynucleotide is characterized by at least one end sequence of SEQ ID NO: 28 and/or SEQ ID NO: 29.

In another specific embodiment, a polynucleotide, particularly an isolated polynucleotide, according to the invention and as described herein before is provided or an informative part or fragment thereof, which polynucleotide is characterized by the end sequence of SEQ ID NO: 23.

in another specific embodiment, a polynucleotide, particularly an isolated polynucleotide, according to the invention and as described herein before is provided or an informative part or fragment thereof, which is further characterized by a DNA fragment that can be amplified in a PCR reaction using primer pair 1 represented by a forward primer of SEQ ID NO: 2 and a reverse primer of SEQ ID NO: 3 and/or 54.

In another specific embodiment, a polynucleotide, particularly an isolated polynucleotide, according to the invention and as described herein before is provided or an informative part or fragment thereof, which is further characterized by
i) a DNA fragment comprising an intronic region that can be obtained from the sugar beet genome by PCR amplification based on forward primer 9_27-F and reverse primer 9_27-R as given in SEQ ID NO: 2 and SEQ ID NO: 3, respectively, particularly a DNA fragment exhibiting a nucleotide sequence as depicted in SEQ ID NOs: 5, or a sequence that has at least 70%, particularly at least 75%, more particularly at least 80%, even more particularly at least 85%, but especially at least 90% and up to at least 95%-99% sequence identity therein;
ii) a polynucleotide fragment comprising a nucleotide sequence which has 70%, particularly 75%, more particularly 80%, even more particularly 85%, but especially 90% and up to 95%-99% sequence identity with a nucleotide sequence depicted in SEQ ID NO: 4;
iii) a polynucleotide fragment which, after splicing, has 70%, particularly 75%, more particularly 80%, even more particularly 85%, but especially 90% and up to 95%-99% sequence identity with a cDNA sequence shown in SEQ ID NO:1.

In another specific embodiment, a polynucleotide, particularly an isolated polynucleotide, according to the invention and as described herein before is provided or an informative part or fragment thereof, which polynucleotide is characterized by at least one end sequence which can be amplified using primer pair 6 represented by a forward primer of SEQ ID NO: 40 and a reverse primer of SEQ ID NO: 41 and/or primer pair 7 represented by a forward primer of SEQ ID NO: 42 and a reverse primer of SEQ ID NO: 43.

In another specific embodiment, a polynucleotide, particularly an isolated polynucleotide, according to the invention and as described herein before is provided or an informative part or fragment thereof, which polynucleotide is characterized by an end sequence which can be amplified using primer pair 8 represented by a forward primer of SEQ ID NO: 44 and a reverse primer of SEQ ID NO: 45.

In another specific embodiment, a polynucleotide, particularly an isolated polynucleotide, according to the invention and as described herein before is provided or an informative part or fragment thereof, which polynucleotide is characterized by at least one end sequence which can be amplified using primer pair 9 represented by a forward primer of SEQ ID NO: 46 and a reverse primer of SEQ ID NO: 47 and/or primer pair 10 represented by a forward primer of SEQ ID NO: 48 and a reverse primer of SEQ ID NO: 49.

In another specific embodiment, a polynucleotide, particularly an isolated polynucleotide, according to the invention and as described herein before is provided or an informative part or fragment thereof, which polynucleotide is characterized by at least one end sequence which can be amplified using primer pair 11 represented by a forward primer of SEQ ID NO: 50 and a reverse primer of SEQ ID NO: 51 and/or primer pair 12 represented by a forward primer of SEQ ID NO: 52 and a reverse primer of SEQ ID NO: 53.

In another specific embodiment, a polynucleotide, particularly an isolated polynucleotide, according to the invention and as described herein before is provided or an informative part or fragment thereof, which polynucleotide is characterized by at least one end sequence of SEQ ID NO: 26 and/or SEQ ID NO: 27.

In another specific embodiment, a polynucleotide including an informative fragment thereof, particularly an isolated polynucleotide, according to the invention and as described herein before is provided or an informative part or fragment thereof, which polynucleotide is characterized by at least one end sequence of SEQ ID NO: 30 and/or SEQ ID NO: 31.

In another specific embodiment, a polynucleotide including an informative fragment thereof, particularly an isolated polynucleotide, according to the invention and as described herein before is provided or an informative part or fragment thereof, which polynucleotide is characterized by at least one end sequence of SEQ ID NO: 32 and/or SEQ ID NO: 33.

In another specific embodiment, a polynucleotide including an informative fragment thereof, particularly an isolated polynucleotide, according to the invention and as described herein before is provided or an informative part or fragment thereof, which polynucleotide is characterized by at least one end sequence of SEQ ID NO: 24 and/or SEQ ID NO: 25.

The polynucleotide, particularly an isolated polynucleotide including an informative fragment thereof, according to the invention and as described herein before can be used for developing a marker for detecting a polymorphism within a genomic region comprising said polynucleotide fragment, particularly a polymorphism that is based on an SNP, an SSR, a deletion or an insertion of at least one nucleotide, but especially a polymorphism based on an SNP, which polymorphism is diagnostic for the *B* allele at the *B* locus and allows to discriminate between the annual and biennial genotype or between different haplotypes within plant groupings of sugar beet plants exhibiting a biennial genotype.

In one embodiment of the invention, the polynucleotide fragment according to the invention, particularly the polynucleotide fragment exhibiting a nucleotide sequence as depicted in SEQ ID NO: 4 and SEQ ID NO: 1, respectively, is genetically linked and thus representative of the bolting gene or B gene.

In one embodiment, the invention relates to one or a plurality of primers, particularly one or a plurality of primer pairs, but especially one or a plurality of primer pairs consisting of a forward primer and a reverse primer, which primers are capable of annealing to a nucleotide sequence within a genomic region of the sugar beet genome that is genetically closely linked to the *B* gene and comprises a polynucleotide according to the invention and as described herein before and of amplifying an informative fragment thereof, wherein said fragment comprises a polymorphism, particularly a polymorphism that is based on an SNP, an SSR, a deletion or an insertion of at least one nucleotide, but especially a polymorphism based on an SNP, which polymorphism is diagnostic for the B allele at the *B* locus and allows to discriminate between the annual and biennial genotype or between different haplotypes within plant groupings of sugar beet plants exhibiting a biennial or annual genotype.

In one embodiment, a pair of primers is provided consisting of a forward primer and a reverse primer which primers are capable of annealing to a nucleotide sequence within a genomic region of the sugar beet genome that is genetically closely linked to the *B* gene and comprises an intronic region, which intronic region is obtainable from the sugar beet genome by PCR amplification based on forward primer 9_27-F and reverse primer 9_27-R as given in SEQ ID NO: 2 and SEQ ID NO: 3, respectively, and of amplifying an informative fragment thereof, wherein said fragment comprises one or more polymorphisms, particularly a polymorphism that is based on an SNP, an SSR, a deletion or an insertion of at least one nucleotide as shown, for example, in Fig. 8, but especially a polymorphism based on an SNP, which polymorphism is diagnostic for the B allele at the B locus and allows to discriminate between the annual and biennial genotype or between different haplotypes within plant groupings of sugar beet plants exhibiting a biennial genotype.

In one embodiment, the intronic region has at least 70%, particularly at least 75%, more particularly at least 80%, even more particularly at least 85%, but especially at least 90% and up to at least 95%-99% sequence identity with the nucleotide sequence depicted in SEQ ID NO: 5.

In a specific embodiment, the intronic region comprises a nucleotide sequence as shown in SEQ ID NO: 5.

In another specific embodiment, the polymorphic site within the intronic region is characterized by a polymorphism based on a SNP, a SSR, a deletion or an insertion of at least one nucleotide as shown, for example, in Fig. 8.

In a specific embodiment, a pair of primers is provided according to the invention and as described herein before, which anneals to a nucleotide sequence within the intronic region depicted in SEQ ID NO: 5 and amplifies an informative fragment from said region comprising a polymorphism, particularly a polymorphism comprising a G/C SNP at position #598 and/or an A/C SNP at position #631.

In particular, said pair of primers comprises a forward primer as depicted in SEQ ID NO: 8 and a reverse primer as depicted in SEQ ID NO: 9 for amplifying a fragment comprising SNP #631, and a forward primer as depicted in SEQ ID NO: 12 and a reverse primer as depicted in SEQ ID NO: 13 for amplifying a fragment comprising SNP #598.

In one embodiment, a pair of primers is provided consisting of a forward primer and a reverse primer which primers are capable of annealing to a nucleotide sequence within a genomic region of the sugar beet genome that is genetically closely linked to the B gene and comprises a nucleotide sequences as depicted in SEQID NO:4.

In one embodiment, a pair of primers is provided consisting of a forward primer and a reverse primer which primers are capable of annealing to a nucleotide sequence within a genomic region of the sugar beet genome that is genetically closely linked to the *B* gene and comprises a nucleotide sequence corresponding to and obtainable from at least one BAC selected from a group of partially overlapping BACs, which BACs can be identified and isolated from a BAC library developed from the biennial commercial cultivar H20 and tiled based on their BAC end sequences (BES) which can be amplified using a primer pair selected from the group consisting of:
- primer pair 2 represented by a forward primer of SEQ ID NO: 34 and a reverse primer of SEQ ID NO: 35,
- primer pair 3 represented by a forward primer of SEQ ID NO: 21 and a reverse primer of SEQ ID NO: 22,
- primer pair 4 represented by a forward primer of SEQ ID NO: 36 and a reverse primer of SEQ ID NO: 37,
- primer pair 5 represented by a forward primer of SEQ ID NO: 38and a reverse primer of SEQ ID NO: 39,
- primer pair 6 represented by a forward primer of SEQ ID NO: 40 and a reverse primer of SEQ ID NO: 41,
- primer pair 7 represented by a forward primer of SEQ ID NO: 42 and a reverse primer of SEQ ID NO: 43,
- primer pair 8 represented by a forward primer of SEQ ID NO: 44 and a reverse primer of SEQ ID NO: 45,
- primer pair 9 represented by a forward primer of SEQ ID NO: 46 and a reverse primer of SEQ ID NO: 47,
- primer pair 10 represented by a forward primer of SEQ ID NO: 48 and a reverse primer of SEQ ID NO: 49,
- primer pair 11 represented by a forward primer of SEQ ID NO: 50 and a reverse primer of SEQ ID NO: 51, and
- primer pair 12 represented by a forward primer of SEQ ID NO: 52 and a reverse primer of SEQ ID NO: 53,
and of amplifying an informative fragment thereof, wherein said fragment comprises one or more polymorphisms, particularly a polymorphism that is based on an SNP, an SSR, a deletion or an insertion of at least one nucleotide, but especially a polymorphism based on an SNP, which polymorphism is diagnostic for the *B* allele at the *B* locus and allows to discriminate between the annual and biennial genotype or between different haplotypes within plant groupings of sugar beet plants exhibiting a biennial genotype.

In a specific embodiment, a pair of primers is provided according to the invention and as described herein before, which anneals to a nucleotide sequence within at least one of the BES regions depicted in any one of SEQ ID NOs: 20 and 23-33 and amplifies an informative fragment from said region comprising a polymorphism, particularly a polymorphism based on SNP.

In another specific embodiment, a pair of primers is provided according to the invention and as described herein before, which anneals to a nucleotide sequence within the BES region depicted in SEQ ID NO: 20 and amplifies an informative fragment from said region comprising a polymorphism, particularly a polymorphism comprising an A/T SNP at position #1179 and an A/C SNP at position 1180.

In particular, said pair of primers comprises a forward primer as depicted in SEQ ID NO: 16 and a reverse primer as depicted in SEQ ID NO: 17 for amplifying a fragment comprising an A/T SNP at position #1179 and an A/C SNP at position 1180.

In one embodiment, the invention relates to a set of probe polynucleotides comprising at least two separate probe molecules that are complementary to a sub-region within an informative polynucleotide fragment according to the invention and as described herein before comprising a polymorphic site and amplify partially overlapping fragments which differ only by one or two base mismatches in the area of overlap, wherein a first probe, particularly a probe labelled with a first fluorescent dye, more particularly with a first fluorescent dye and a quencher represents one allele and a second probe, particularly a probe labelled with a second fluorescent dye, which is not identical with the first dye, more particularly with a second fluorescent dye and a quencher, represents the other allele.

In a specific embodiment of the invention, said informative polynucleotide fragment comprises a polymorphism, wherein said polymorphism is based on SNP #631 within the intronic region depicted in SEQ ID NO: 5 and the first probe molecule labelled with a first fluorescent dye, has a nucleotide sequence as shown in SEQ ID NO:10 and the second probe molecule labelled with a second fluorescent dye, has a nucleotide sequence as shown in SEQ ID NO:11.

In another specific embodiment of the invention, said informative polynucleotide fragment comprises a polymorphism, wherein said polymorphism is based on SNP #598 within the intronic region depicted in SEQ ID NO: 5 and the first probe molecule labelled with a first fluorescent dye, has a nucleotide sequence as shown in SEQ ID NO:12 and the second probe molecule labelled with a second fluorescent dye, has a nucleotide sequence as shown in SEQ ID NO:13.

In another specific embodiment of the invention, said informative polynucleotide fragment comprises a polymorphism, wherein said polymorphism is based on SNP #1179/1180 within the BES region depicted in SEQ ID NO: 18 and the first probe molecule labelled with a first fluorescent dye, has a nucleotide sequence as shown in SEQ ID NO:18 and the second probe molecule labelled with a second fluorescent dye, has a nucleotide sequence as shown in SEQ ID NO:19.

In one embodiment, the invention relates to the use of a polynucleotide according to the invention and as described herein before, or any informative fragment thereof, for developing a marker that may be used in an allelic discrimination assay for detecting a polymorphism in the sugar beet genome, which polymorphism is diagnostic for the *B* allele at the B locus and allows to discriminate between the annual and biennial genotype or between different haplotypes within plant groupings of sugar beet plants exhibiting a biennial genotype or for mapping the *B*-gene to the sugar beet genome.

In a specific embodiment, the invention relates to the use of one or a plurality of primers, particularly one or a plurality of primer pairs, according to the invention and as described herein before in an allelic discrimination assay for detecting a polymorphism in the sugar beet genome, particularly a polymorphism that is based on an SNP, an SSR, a deletion or an insertion of at least one nucleotide, but especially a polymorphism based on an SNP, which polymorphism is diagnostic for the *B* allele at the *B* locus and allows to discriminate between the annual and biennial genotype or between different haplotypes within plant groupings of sugar beet plants exhibiting a biennial genotype.

In another specific embodiment of the invention, a set of probe molecules according to the invention and as described herein before may in addition be employed in said allelic discrimination assay.

In one embodiment, the invention relates to a method of identifying the absence or presence of an allele associated with annuality in a sugar beet plant comprising
a) obtaining a genomic sample from a sugar beet plant to be analyzed,
b) analyzing the nucleotide sequence of the genomic region of the sugar beet genome that is genetically closely linked to the *B* gene and complementary to or comprises the sequence of a polynucleotide according to the invention and as described herein before, and
c) comparing said sequence with an acetic sequence known to be associated with the biennial phenotype and the annual phenotype, respectively,

In one embodiment, the invention relates to a method of identifying a specific haplotype within a plant grouping of sugar beet plants exhibiting a biennial genotype comprising
a) obtaining a genomic sample from a sugar beet plant to be analyzed,
b) analyzing the nucleotide sequence of the genomic region of the sugar beet genome that is genetically closely linked to the *B* gene and complementary to or comprises the sequence of a polynucleotide according to the invention and as described herein before, and
c) comparing said sequence with an allelic sequence known to be associated with a specific haplotype.

In a specific embodiment, the sequence analysis is carried out using a molecular marker based on a polynucleotide or an informative fragment thereof or on one or a plurality of primers, particularly on one or a plurality of primer pairs, but especially on one or a plurality of primer pairs consisting of a forward primer and a reverse primer according to the invention and as described herein before.

In another specific embodiment, a method of identifying the absence or presence of an allele associated with annuality in a sugar beet plant is provided comprising
a) obtaining a genomic sample from a sugar beet plant to be analyzed,
b) analyzing the nucleotide sequence of an intronic region obtainable from the sugar beet genome by PCR amplification based on forward primer 9_27-F and reverse primer 9_27-R as given in SEQ ID NO:2 and SEQ ID NO: 3, and
c) comparing said sequence with an allelic sequence known to be associated with the biennial phenotype and the annual phenotype, respectively.

In one embodiment, the intronic region has at least 70%, particularly at least 75%, more particularly at least 80%, even more particularly at least 85%, but especially at least 90% and up to at least 95%-99% sequence identity with the nucleotide sequence depicted in SEQ ID NO: 5.

In still another specific embodiment the intronic region has a nucleotide sequence as shown in SEQ ID NO: 5.

In another specific embodiment, a method of identifying the absence or presence of an allele associated with annuality in a sugar beet plant is provided comprising
a) obtaining a genomic sample from a sugar beet plant to be analyzed,
b) analyzing the nucleotide sequence of a genomic region comprising a nucleotide sequence as given in SEQ ID NO: 4, and
c) comparing said sequence with an allelic sequence known to be associated with the biennial phenotype and the annual phenotype, respectively.

In another specific embodiment, a method of identifying the absence or presence of an allele associated with annuality in a sugar beet plant is provided comprising
a) obtaining a genomic sample from a sugar beet plant to be analyzed,
b) analyzing the nucleotide sequence of a genomic region comprising a nucleotide sequence corresponding to and obtainable from at least one BAC selected from a group of partially overlapping BACs, which BACs can be identified and isolated from a BAC library developed from the biennial commercial cultivar H20 and tiled based on their BAC end sequences (BES) which can be amplified using a primer pair selected from the group consisting of:
   - primer pair 2 represented by a forward primer of SEQ ID NO: 34 and a reverse primer of SEQ ID NO: 35,
   - primer pair 3 represented by a forward primer of SEQ ID NO: 21 and a reverse primer of SEQ ID NO: 22,
   - primer pair 4 represented by a forward primer of SEQ ID NO: 36 and a reverse primer of SEQ ID NO: 37,
   - primer pair 5 represented by a forward primer of SEQ ID NO: 38and a reverse primer of SEQ ID NO: 39,
   - primer pair 6 represented by a forward primer of SEQ ID NO: 40 and a reverse primer of SEQ ID NO: 41,
   - primer pair 7 represented by a forward primer of SEQ ID NO: 42 and a reverse primer of SEQ ID NO: 43,
   - primer pair 8 represented by a forward primer of SEQ ID NO: 44 and a reverse primer of SEQ ID NO: 45,
   - primer pair 9 represented by a forward primer of SEQ ID NO: 46 and a reverse primer of SEQ ID NO: 47,
   - primer pair 10 represented by a forward primer of SEQ ID NO: 48 and a reverse primer of SEQ ID NO: 49,
   - primer pair 11 represented by a forward primer of SEQ ID NO: 50 and a reverse primer of SEQ ID NO: 51, and
   - primer pair 12 represented by a forward primer of SEQ ID NO: 52 and a reverse primer of SEQ ID NO: 53
c) comparing one of more of the amplified sequences with an allelic sequence known to be associated with the biennial phenotype and the annual phenotype, respectively.

In a specific embodiment of the invention said BES region has a nucleotide sequence of any one of the sequences depicted in SEQ ID NOs: 23-33.

In still another specific embodiment, a method is provided wherein within a genomic sample from a sugar beet plant the intronic region of a polynucleotide according to the invention and as described herein before is analyzed using a forward and a reverse primer flanking a sub-region within said intronic region known to comprise a polymorphic site, amplifying said sub-region and comparing the amplified fragment with an allelic sequence known to be associated with the biennial phenotype and the annual phenotype, respectively.

In particular, a forward primer and a reverse primer are used exhibiting a nucleotide sequence as shown in SEQ ID NO: 8 and SEQ ID NO: 9, respectively.

In another specific embodiment, a forward primer and a reverse primer are used exhibiting a nucleotide sequence as shown in SEQ ID NO: 12 and SEQ ID NO: 13, respectively.

In another specific embodiment, a forward primer and a reverse primer are used exhibiting a nucleotide sequence as shown in SEQ ID NO: 16 and SEQ ID NO: 17, respectively.

In another specific embodiment, a method is provided as described herein before, wherein a set of probe polynucleotides is designed based on said SNP comprising two separate probe molecules which differ by at least one mismatch, particularly by two or more mismatches located at adjacent sites, but especially by one single mismatch, wherein a first probe molecule, particularly a labelled probe molecule, more particularly a probe molecule labelled with a first fluorescent dye and a quencher, represents one allele and a second probe molecule, particularly a labelled probe molecule, more particularly a probe molecule labelled with a second fluorescent dye and a quencher, which is not identical with the first dye, represents the other allele, and wherein said set of probe polynucleotides is used for discriminating between the two allelic variants.

In one embodiment, an allelic discrimination assay is provided for detecting a polymorphism in a genomic region of the sugar beet genome co-segregating with the annuality phenotype, particularly a polymorphism that is based on an SNP, an SSR, a deletion or an insertion of at least one nucleotide, but especially a polymorphism based on an SNP, which polymorphism is diagnostic for the *B* allele at the *B* locus and allows to discriminate between the annual and biennial genotype, comprising a molecular marker based on a polynucleotide according to the invention and as described herein before or any informative fragment thereof.

In a specific embodiment, said molecular marker comprises a pair of primers according to the invention and as described herein before.

In another specific embodiment, an allelic discrimination assay is provided for detecting a single-base polymorphism in an intronic region obtainable from the sugar beet genome by PCR amplification based on forward primer 9_27-F and reverse primer 9_27-R as given in SEQ ID NO: 2 and SEQ ID NO: 3, comprising a set of primers and/or probe polynucleotides according to the invention and as described herein before.

In one embodiment, the intronic region has at least 70%, particularly at least 75%, more particularly at least 80%, even more particularly at least 85%, but especially at least 90% and up to at least 95%-99% sequence identity with the nucleotide sequence depicted in SEQ ID NO: 5.

In a specific embodiment, the intronic region comprises a nucleotide sequence as shown in SEQ ID NO: 5.

In another specific embodiment, an allelic discrimination assay is provided for detecting a single-base polymorphism in genomic region of the sugar beet genome that is genetically closely linked to the *B* gene and comprises a nucleotide sequences as depicted in SEOID NO:4, comprising a set of primers and/or probe polynucleotides according to the invention and as described herein before, which primers and/or probe polynucleotides are capable of amplifying an informative fragment from said region comprising a polymorphism, particularly a polymorphism based on SNP.

in another specific embodiment, an allelic discrimination assay is provided for detecting a single-base polymorphism in genomic region of the sugar beet genome that is genetically closely linked to the *B* gene and comprises a nucleotide sequence corresponding to and obtainable from at least one BAC selected from a group of partially overlapping BACs, which BACs can be identified and isolated from a BAC library developed from the biennial commercial cultivar H20 and tiled based on their BAC end sequences (BES) which can be amplified using a primer pair selected from the group consisting of:
- primer pair 2 represented by a forward primer of SEQ ID NO: 34 and a reverse primer of SEQ ID NO: 35,
- primer pair 3 represented by a forward primer of SEQ ID NO: 21 and a reverse primer of SEQ ID NO: 22,
- primer pair 4 represented by a forward primer of SEQ ID NO: 36 and a reverse primer of SEQ ID NO: 37,
- primer pair 5 represented by a forward primer of SEQ ID NO: 38and a reverse primer of SEQ ID NO: 39,
- primer pair 6 represented by a forward primer of SEQ ID NO: 40 and a reverse primer of SEQ ID NO: 41,
- primer pair 7 represented by a forward primer of SEQ ID NO: 42 and a reverse primer of SEQ ID NO: 43,
- primer pair 8 represented by a forward primer of SEQ ID NO: 44 and a reverse primer of SEQ ID NO: 45,
- primer pair 9 represented by a forward primer of SEQ ID NO: 46 and a reverse primer of SEQ ID NO: 47,
- primer pair 10 represented by a forward primer of SEQ ID NO: 48 and a reverse primer of SEQ ID NO: 49,
- primer pair 11 represented by a forward primer of SEQ ID NO: 50 and a reverse primer of SEQ ID NO: 51, and
- primer pair 12 represented by a forward primer of SEQ ID NO: 52 and a reverse primer of SEQ ID NO: 53,
comprising a set of primers and/or probe polynucleotides according to the invention and as described herein before, which primers and/or probe polynucleotides are capable of annealing to said nucleotide sequence and of amplifying an informative fragment thereof, wherein said fragment comprises one or more polymorphisms, particularly a polymorphism that is based on an SNP, an SSR, a deletion or an insertion of at least one nucleotide, but especially a polymorphism based on an SNP, which polymorphism is diagnostic for the B allele at the *B* locus and allows to discriminate between the annual and biennial genotype.

In one embodiment, the invention relates to the use of a polynucleotide according to the invention and as described herein before for the development of a molecular marker to be used for identifying the absence or presence of an allele associated with annuality in a sugar beet genome comprising
a) identifying in said polynucleotide polymorphic sites
b) associating said polymorphisms with the absence or presence of an allele associated with annuality in sugar beet by
c) designing a primer or a plurality of primers, particularly a pair of primers or a plurality of primer pairs, but especially a forward and reverse primer recognizing a nucleotide sequence flanking this polymorphic site that can be used in an allelic discrimination assay.

In one embodiment, the invention relates to a method of identifying annual contaminations in commercial seed using a polynucleotide according to the invention and as described herein before or an informative fragment thereof as a marker for determining the presence or absence of the annuality allele in a plant sample.

In particular, the invention relates to a method of identifying annual contaminations in commercial seed using a polynucleotide according to the invention and as described herein before or an informative fragment thereof as a marker for identifying annual contaminations in commercial seed.

In one embodiment, the invention relates to a method of identifying annual contaminations in commercial seed using a marker-based allelic discrimination assay according to the invention and as described herein before.

### Brief Description of the Figures and Sequences

### FIGURES

Figure 1 shows the cDNA sequence of the 9_27 gene fragment; the nucleotides sequences formatted in bold and italic indicate the forward 9_27-F (GCCATTTGGTGATACGACTC) and reverse 9_27-R (TGCTAGGTTGCAGTTTGCT) primers used for PCR amplification.

Figure 2 shows the alignment of RMM domains from different species. Species names are abbreviated as follows; Bv, Beta vulgaris; Hs, Homo sapiens; Ce, Caenorhabditis elegans; Sp, Schizosaccharomyces pombe; At, Arabidopsis thaliana; Gg, Gallus gallus. Numbers flanking the abbreviations indicate the accession numbers.

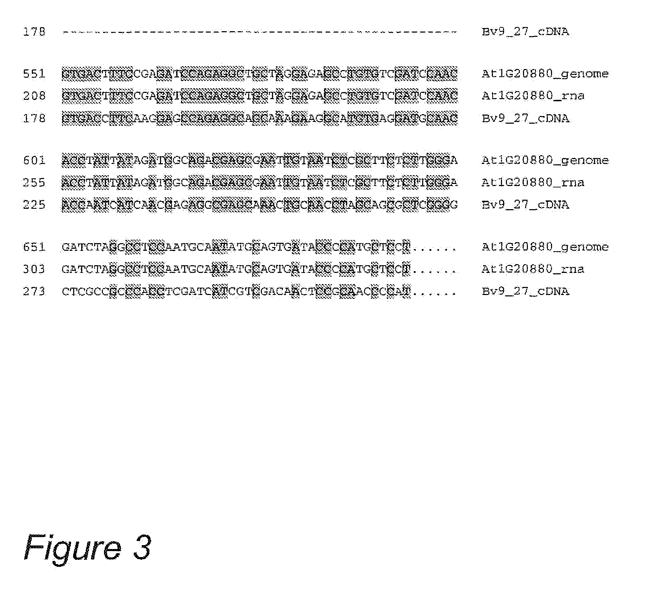

Figure 3 shows the determination of the putative gene structure of 9_27 gene fragment. 9_27 cDNA and At1G20880 mRNA sequence were aligned against the At1G20880 genomic sequence. Only the region harbouring the RRM domain is shown. Intron #1 from At1G20880 is printed in light grey.

Figure 4 shows the genomic sequence of the 9_27 gene fragment; the bold/italic formatted sequences indicate the 9_27-F and 9_27-R primer pair. The sequence printed in italic indicates the intronic region.

Figure 5 shows the map of the chromosome II of the sugar beet crop. The genetic distances at the left side are expressed in cM.

Figure 6 shows the BES ED031700 sequence; the bold/italic formatted sequences indicate the forward ED031700-F (CCTTTTAGACCTGAGCATGG) and reverse ED031700-R (AGAGATTGGGGCGGAATAG) primers used for PCR amplification.

Figure 7 shows the BAC contig comprising the 9_27 gene fragment. Horizontal lines represent individual BAC clones, vertical lines represent PCR assays derived from the BES sequences that were used to tile the BAC clones.

Figure 8 shows the_polymorphisms observed for the complete intronic region 1 of the 9_27 gene

### SEQUENCES

SEQ ID NO:1 depicts the nucleotide sequence of the 9_27 cDNA fragment
SEQ ID NO:2 depicts the nucleotide sequence of forward primer 9_27-F
SEQ ID NO:3 depicts the nucleotide sequence of reverse primer 9_27-R
SEQ ID NO:4 depicts the nucleotide sequence of a genomic *Beta vulgaris* DNA fragment
SEQ ID NO:5 depicts the nucleotide sequence of an intronic region
SEQ ID NO:6 depicts the putative protein sequence from the 5' coding region of the 9_27 gene
SEQ ID NO:7 depicts the amino acid sequence of the putative conserved RRM domain
SEQ ID NO:8 depicts the nucleotide sequence of forward primer 9_27(T1)-F
SEQ ID NO:9 depicts the nucleotide sequence of reverse primer 9_27(T1)-R
SEQ ID NO:10 depicts the nucleotide sequence of primer 9_27(T1)-FAM
SEQ ID NO:11 depicts the nucleotide sequence of primer 9_27(T1)-VIC
SEQ ID NO:12 depicts the nucleotide sequence of forward primer 9_27(T2)-F
SEQ ID NO:13 depicts the nucleotide sequence of reverse primer 9_27(T2)-R
SEQ ID NO:14 depicts the nucleotide sequence of primer 9_27(T2)-FAM
SEQ ID NO:15 depicts the nucleotide sequence of primer 9_27(T2)-VIC
SEQ ID NO:16 depicts the nucleotide sequence of forward primer ED031700 (T1)-F
SEQ ID NO:17 depicts the nucleotide sequence of reverse primer ED031700 (T1)-R
SEQ ID NO:18 depicts the nucleotide sequence of primer ED031700 (T1)-FAM
SEQ ID NO:19 depicts the nucleotide sequence of primer ED031700 (T1)-VIC
SEQ ID NO:20 depicts the nucleotide sequence of BAC end ED031700
SEQ ID NO:21 depicts the nucleotide sequence of forward primer ED031700-F
SEQ ID NO:22 depicts the nucleotide sequence of reverse primer ED031700-R
SEQ ID NO:23 depicts the nucleotide sequence of BAC end SBA039-123_T7
SEQ ID NO:24 depicts the nucleotide sequence of BAC end SBA049-A21_Sp6
SEQ ID NO:25 depicts the nucleotide sequence of BAC end SBA049-A21_T7
SEQ ID NO:26 depicts the nucleotide sequence of BAC end SBA066-E16_Sp6
SEQ ID NO:27 depicts the nucleotide sequence of BAC end SBA066-E16_T7
SEQ ID NO:28 depicts the nucleotide sequence of BAC end SBA071-P24_Sp6
SEQ ID NO:29 depicts the nucleotide sequence of BAC end S8A071-P24_T7
SEQ ID NO:30 depicts the nucleotide sequence of BAG end SBA079-O22_Sp6
SEQ ID NO:31 depicts the nucleotide sequence of BAG end SBA079-O22_T7
SEQ ID NO:32 depicts the nucleotide sequence of BAC end SBA083-N13_Sp6
SEQ ID NO:33 depicts the nucleotide sequence of BAC end SBA083-N13_T7
SEQ ID NO:34 depicts the nucleotide sequence of forward primer HiNK6986
SEQ ID NO:35 depicts the nucleotide sequence of reverse primer HiNK6989
SEQ ID NO:36 depicts the nucleotide sequence of forward primer HiNK7534
SEQ ID NO:37 depicts the nucleotide sequence of reverse primer HiNK7536
SEQ ID NO:38 depicts the nucleotide sequence of forward primer HiNK7538
SEQ ID NO:39 depicts the nucleotide sequence of reverse primer HiNK7539
SEQ ID NO:40 depicts the nucleotide sequence of forward primer HiNK7563
SEQ ID NO:41 depicts the nucleotide sequence of reverse primer HiNK7565
SEQ ID NO:42 depicts the nucleotide sequence of forward primer HiNK7766
SEQ ID NO:43 depicts the nucleotide sequence of reverse primer HiNK7768
SEQ ID NO:44 depicts the nucleotide sequence of forward primer HiNK7543
SEQ ID NO:45 depicts the nucleotide sequence of reverse primer HiNK7545
SEQ ID NO:46 depicts the nucleotide sequence of forward primer HiNK7550
SEQ ID NO:47 depicts the nucleotide sequence of reverse primer HiNK7552
SEQ ID NO:48 depicts the nucleotide sequence of forward primer HiNK7554
SEQ ID NO:49 depicts the nucleotide sequence of reverse primer HiNK7556
SEQ ID NO:50 depicts the nucleotide sequence of forward primer HiNK7522
SEOIO NO:51 depicts the nucleotide sequence of reverse primer HiNK7525
SEQ ID NO:52 depicts the nucleotide sequence of forward primer HiNK7528
SEQ ID NO:53 depicts the nucleotide sequence of reverse primer HiNK7532
SEQ ID NO:54 depicts the nucleotide sequence of reverse primerHiNK6853
SEQ ID NO:55 depicts the nucleotide sequence of the intronic region comprising SNP#631
SEQ ID NO:56 depicts the nucleotide sequence of the intronic region comprising SNP#598
SEQ ID NO:57 depicts the nucleotide sequence of BES ED031700comprising SNP #1179/1180

### DEFINITIONS

The technical terms and expressions used within the scope of this application are generally to be given the meaning commonly applied to them in the pertinent art of plant molecular biology if not otherwise indicated herein below.

As used in this specification and the appended claims, the singular forms "a", "an", and "the" include plural referents unless the context clearly dictates otherwise. Thus, for example, reference to "a plant" includes one or more plants, and reference to "a cell" includes mixtures of cells, tissues, and the like.

"Sugar beet" refers to all species and subspecies within the genus *Beta* as well as all kinds of cultivated beets of *Beta vulgaris.* Cultivated beets have been separated into four groups: leaf beet, garden beet, fodder beet and sugar beet. "Sugar beet" refers also to all cultivated beets including those grown for other purposes than the production of sugar, such as ethanol, plastics or industrial products. In particular, "Sugar beet" refers to fodder beet and sugar beet, but especially to sugar beet.

An "annual sugar beet line" refers to a sugar beet plant containing the dominant allele *B* at the *B* locus in a heterozygous or homozygous state.

A "biennial sugar beet line" refers to a sugar beet plant containing the recessive allele *B* at the *B* locus in a homozygous state

"Bolting" refers to the transition from the vegetative rosette stage to the inflorescence or reproductive growth stage.

"Vernalization" refers to the process by which floral induction in some plants is promoted by exposing the plants to chilling for certain duration.

An "allele" is understood within the scope of the invention to refer to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes.

As used herein, the term "breeding", and grammatical variants thereof, refer to any process that generates a progeny individual. Breedings can be sexual or asexual, or any combination thereof. Exemplary non-limiting types of breedings include crossings, selfings, doubled haploid derivative generation, and combinations thereof.

"Locus" is understood within the scope of the invention to refer to a region on a chromosome, which comprises a gene or any other genetic element or factor contributing to a trait.

As used herein, the phrase "genetic marker" refers to a feature of an individual's genome (*e.g.,* a nucleotide or a polynucleotide sequence that is present in an individual's genome) that is associated with one or more loci of interest. In some embodiments, a genetic marker is polymorphic in a population of interest, or the locus occupied by the polymorphism, depending on context. Genetic markers include, for example, single nucleotide polymorphisms (SNPs), indels (*i.e.,* insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples. Genetic markers can, for example, be used to locate genetic loci containing alleles that contribute to variability in expression of phenotypic traits on a chromosome. The phrase "genetic marker" can also refer to a polynucleotide sequence complementary to a genomic sequence, such as a sequence of a nucleic acid used as probes.

A genetic marker can be physically located in a position on a chromosome that is within or outside of to the genetic locus with which it is associated (*i.e.,* is intragenic or extragenic, respectively). Stated another way, whereas genetic markers are typically employed when the location on a chromosome of the gene that corresponds to the locus of interest has not been identified and there is a non-zero rate of recombination between the genetic marker and the locus of interest, the presently disclosed subject matter can also employ genetic markers that are physically within the boundaries of a genetic locus (*e.g.,* inside a genomic sequence that corresponds to a gene such as, but not limited to a polymorphism within an intron or an exon of a gene). In some embodiments of the presently disclosed subject matter, the one or more genetic markers comprise between one and ten markers, and in some embodiments the one or more genetic markers comprise more than ten genetic markers.

The term "polynucleotide" is understood herein to refer to polymeric molecule of high molecular weight which can be single-stranded or double-stranded, composed of monomers (nucleotides) containing a sugar, phosphate and a base which is either a purine or pyrimidine. A "polynucleotide fragment" is a fraction of a given polynucleotide molecule. In higher plants, deoxyribonucleic acid (DNA) is the genetic material while ribonucleic acid (RNA) is involved in the transfer of information contained within DNA into proteins. A "genome" is the entire body of genetic material contained in each cell of an organism. The term "polynucleotide " thus refers to a polymer of DNA or RNA which can be single- or double-stranded, optionally containing synthetic, non-natural or altered nucleotide bases capable of incorporation into DNA or RNA polymers. Unless otherwise indicated, a particular nucleic acid sequence of this invention also implicitly encompasses conservatively modified variants thereof (e.g. degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer, et al., Nucleic Acid Res. 19:5081 (1991); Ohtsuka, et al., J. Biol. Chem. 260:2605-2608 (1985); and Rossolini, et al., Mol. Cell. Probes 8:91-98 (1994)). The term polynucleotide is used interchangeably with nucleic acid, nucleotide sequence and may include genes, cDNAs, and mRNAs encoded by a gene, etc.

The polynucleotide of the invention is understood to be provided in isolated form. The term "isolated" means that the polynucleotide disclosed and claimed herein is not a polynucleotide as it occurs in its natural context, if it indeed has a naturally occurring counterpart. Accordingly, the other compounds of the invention described further below are understood to be isolated. If claimed in the context of a plant genome, the polynucleotide of the invention is distinguished over naturally occurring counterparts by the insertion side in the genome and the flanking sequences at the insertion side.

As used herein, the phrase "informative part or fragment " refers to a part or fragment of a polynucleotide with an information content that is retrievable and can assist in the determination and/or characterization of a genetic locus of interest. This information content may be represented by a polymorphism which is associated with said locus of interest such as, for example, a single nucleotide polymorphisms (SNPs), indels (*i.e.*, insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DArT) markers, and amplified fragment length polymorphisms (AFLPs), among many other examples and may be used for the development of a genetic marker. The information content of such an "informative fragment" may also be represented by a specific sequence that can be detected by a corresponding probe molecule.

As used herein, the phrase "nucleic acid" refers to any physical string of monomer units that can be corresponded to a string of nucleotides, including a polymer of nucleotides (*e.g*., a typical DNA or RNA polymer), modified oligonucleotides (*e.g*., oligonucleotides comprising bases that are not typical to biological RNA or DNA, such as 2'-*O*-methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

As used herein, the phrase "phenotypic trait" refers to the appearance or other detectable characteristic of an individual resulting from the interaction of its genome with the environment.

"Marker-based selection" is understood within the scope of the invention to refer to the use of genetic markers to detect one or more nucleic acids from the plant, where the nucleic acid is associated with a desired trait to identify plants that carry genes for desirable (or undesirable) traits, so that those plants can be used (or avoided) in a selective breeding program.

"Microsatellite or SSRs (Simple sequence repeats) (Marker)" is understood within the scope of the invention to refer to a type of genetic marker that consists of numerous repeats of short sequences of DNA bases, which are found at loci throughout the plant's DNA and have a likelihood of being highly polymorphic.

"PCR (Polymerase chain reaction)" is understood within the scope of the invention to refer to a method of producing relatively large amounts of specific regions of DNA, thereby making possible various analyses that are based on those regions.

"PCR primer" is understood within the scope of the invention to refer to rrelatively short fragments of single-stranded DNA used in the PCR amplification of specific regions of DNA.

"Phenotype" is understood within the scope of the invention to refer to a distinguishable characteristic(s) of a genetically controlled trait.

"Polymorphism" is understood within the scope of the invention to refer to the presence in a population of two or more different forms of a gene, genetic marker, or inherited trait.

"Selective breeding" is understood within the scope of the invention to refer to a program of breeding that uses plants that possess or display desirable traits as parents.

The term "hybridize" as used herein refers to conventional hybridization conditions, preferably to hybridization conditions at which 5xSSPE, 1% SDS, 1xDenhardts solution is used as a solution and/or hybridization temperatures are between 35°C and 70°C, preferably 65°C. After hybridization, washing is preferably carried out first with 2xSSC, 1% SDS and subsequently with 0.2xSSC at temperatures between 35°C and 75°C, particularly between 45°C and 65°C, but especially at 59°C (regarding the definition of SSPE, SSC and Denhardts solution see Sambrook et al. loc. cit.). High stringency hybridization conditions as for instance described in Sambrook et al, supra, are particularly preferred. Particularly preferred stringent hybridization conditions are for instance present if hybridization and washing occur at 65°C as indicated above. Non-stringent hybridization conditions for instance with hybridization and washing carried out at 45°C are less preferred and at 35°C even less.

"Sequence Homology or Sequence Identity" is used herein interchangeably. The terms "identical" or percent "identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. If two sequences which are to be compared with each other differ in length, sequence identity preferably relates to the percentage of the nucleotide residues of the shorter sequence which are identical with the nucleotide residues of the longer sequence. Sequence identity can be determined conventionally with the use of computer programs such as the Bestfit program (Wisconsin Sequence Analysis Package, Version 8 for Unix, Genetics Computer Group, University Research Park, 575 Science Drive Madison, WI 53711). Bestfit utilizes the local homology algorithm of Smith and Waterman, Advances in Applied Mathematics 2 (1981), 482-489, in order to find the segment having the highest sequence identity between two sequences. When using Bestfit or another sequence alignment program to determine whether a particular sequence has for instance 95% identity with a reference sequence of the present invention, the parameters are preferably so adjusted that the percentage of identity is calculated over the entire length of the reference sequence and that homology gaps of up to 5% of the total number of the nucleotides in the reference sequence are permitted. When using Bestfit, the socalled optional parameters are preferably left at their preset ("default") values. The deviations appearing in the comparison between a given sequence and the above-described sequences of the invention may be caused for instance by addition, deletion, substitution, insertion or recombination. Such a sequence comparison can preferably also be carried out with the program "fasta20u66" (version 2.0u66, September 1998 by William R. Pearson and the University of Virginia; see also W.R. Pearson (1990), Methods in Enzymology 183, 63-98, appended examples and http://workbench.sdsc.edu/). For this purpose, the "default" parameter settings may be used.

Another indication that two nucleic acid sequences are substantially identical is that the two molecules hybridize to each other under stringent conditions. The phrase: "hybridizing specifically to" refers to the binding, duplexing, or hybridizing of a molecule only to a particular nucleotide sequence under stringent conditions when that sequence is present in a complex mixture (e.g., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. An extensive guide to the hybridization of nucleic acids is found in Tijssen (1993) Laboratory Techniques in Biochemistry and Molecular Biology-Hybridization with Nucleic Acid Probes part I chapter 2 "Overview of principles of hybridization and the strategy of nucleic acid probe assays" Elsevier, New York. Generally, highly stringent hybridization and wash conditions are selected to be about 5 degree C. lower than the thermal melting point (T.sub.m) for the specific sequence at a defined ionic strength and pH. Typically, under "stringent conditions" a probe will hybridize to its target subsequence, but to no other sequences.

The T.sub.m is the temperature (under defined ionic strength and pH) at which 50% of the target sequence hybridizes to a perfectly matched probe. Very stringent conditions are selected to be equal to the T.sub.m for a particular probe. An example of stringent hybridization conditions for hybridization of complementary nucleic acids which have more than 100 complementary residues on a filter in a southern or northern blot is 50% formamide with 1 mg of heparin at 42 degree C., with the hybridization being carried out overnight. An example of highly stringent wash conditions is 0.1 5M NaCl at 72 degree C. for about 15 minutes. An example of stringent wash conditions is a 0.2 times SSC wash at 65 degree C. for 15 minutes (see Sambrook, infra, for a description of SSC buffer). Often, a high stringency wash is preceded by a low stringency wash to remove background probe signal. An example medium stringency wash for a duplex of, e.g., more than 100 nucleotides, is 1 time SSC at 45 degree C. for 15 minutes. An example low stringency wash for a duplex of, e.g., more than 100 nucleotides, is 4-6 times SSC at 40 degree C. for 15 minutes. For short probes (e.g., about 10 to 50 nucleotides), stringent conditions typically involve salt concentrations of less than about 1.0M Na ion, typically about 0.01 to 1.0 M Na ion concentration (or other salts) at pH 7.0 to 8.3, and the temperature is typically at least about 30.degree. C. Stringent conditions can also be achieved with the addition of destabilizing agents such as formamide. In general, a signal to noise ratio of 2 times (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, e.g., when a copy of a nucleic acid is created using the maximum codon degeneracy permitted by the genetic code.

A "plant" is any plant at any stage of development, particularly a seed plant.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

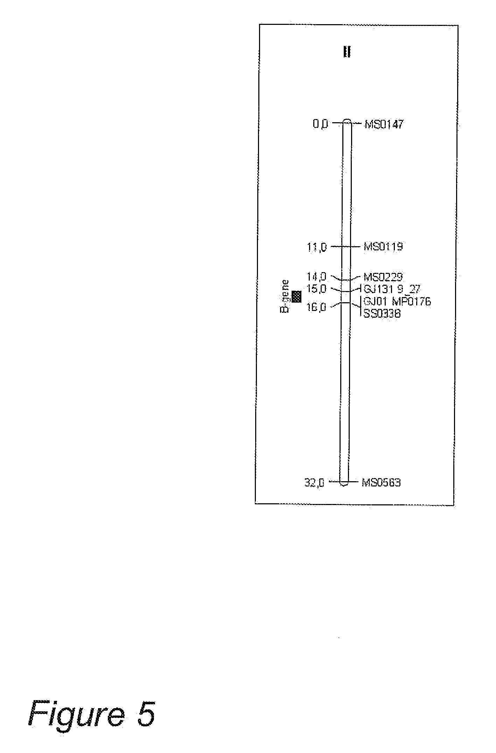

The present invention discloses polynucleotides identified in the sugar beet genome including variants and derivatives thereof and the use of said polynucleotides for the development of markers that can be used for mapping and identification of the bolting gene or *B* gene. The polynucleotide markers according to the invention may also be used for quality control of commercial seed lots by screening of commercial biennial sugar beet seed for annual contaminants and for identifying annuals/biennials in breeding programs, which use the annual trait to speed up the breeding process, or when the annual trait is introduced together with new sources of genetic variation.
The polynucleotides according to the invention and described herein before, can further be used in a transgenic approach for producing transgenic sugar beet plants comprising said polynucleotides stably integrated into the sugar beet genome. In particular, upon expression from the genome, the expression product can be used to modulate the vernalization response of the sugar beet plant.
In one aspect of the invention the vernalization response will be delayed by suppressing or down-regulating expression of the *B*-gene.
In another aspect of the invention, early bolting without cold treatment will be induced upon overexpression of the *B*-gene.
The present invention provides a polynucleotide which maps at or in close vicinity to the B locus, particularly at a distance of 1 cM upstream of markers MP0176 and
GJ01 and co-segregates with marker GJ131 (Möhring S. et al, 2004; Gaafar R. M. et al, 2005) (Figure 5).
In one embodiment, the invention relates to a polynucleotide including an informative fragment thereof according to the invention and as described herein before, which is obtainable from a genomic DNA region that maps at a distance of less than 1 cM, particularly of less than 0.75 cM, more particularly of less than 0.5 cM, even more particularly of less than 0.3 cM, but especially of less than 0.25 cM relative to the B gene.

In particular, the polynucleotide according to the invention comprises a nucleotide sequence that has at least 70%, particularly at least 75%, more particularly at least 80%, even more particularly at least 85%, but especially at least 90% and up to at least 95%-99% sequence identity with the nucleotide sequence depicted in SEQ ID NO:4.
The polynucleotide according to the invention is further characterized by an intronic region that can be obtained from the sugar beet genome by PCR amplification based on forward primer 9_27-F and reverse primer 9_27-R as given in SEQ ID NO: 2 and SEQ ID NO: 3, respectively. This intronic region is further characterized by a length of about 610 base pairs and a high amount of polymorphisms selected from the group of SSRs, SNPs, deletions and insertions of at least 1 and up to 10 to 50 nucleotides as shown in Figure 8. In one embodiment of the invention, the intronic region according to the invention has a nucleotide sequence which is at least 70%, particularly at least 80%, more particularly at least 90%, but especially at least 92%-95% and up to 98%-99% identical to the sequence depicted in SEQ ID NO:5. In another embodiment of the invention, the intronic region has a nucleotide sequence as depicted in Figure 8.
The polynucleotide according to the invention and described herein before comprises a nucleotide sequence which is at least 70%, particularly at least 80%, more particularly at least 90%, but especially at least 92%-95% and up to 98%-99% identical to the sequence depicted in SEQ ID NO: 4.
The polynucleotide according to the invention and describe herein before is further characterized by its encoded polypeptide, which exhibits an amino acid sequence which is at least 70%, particularly at least 80%, more particularly at least 90%, but especially at least 92%-95% and up to 98%-99% identical to the sequence depicted in SEQ ID NO:6.
The polypeptide encoded by the polynucleotide according to the invention contains a RNA Recognition Motif (RRM) conserved domain in the N-terminal region of the peptide.
The polynucleotide according to the invention can further be used to fully characterize the region around the B locus including the B gene and to identify further putative flowering time control candidate genes.
A BAC library has been established with DNA from the biennial commercial sugar beet cultivar H20. Partially (HindIII) digested HMW DNA of fragments in the size of 100-400 kb were size selected two times. The DNA fragments were ligated into the vector pBeloBAC-Kan. The library contains 57,600 clones with an average insert size of approximately 120 kb, corresponding to an 8x coverage of the beet genome. The redundancy has been tested by screening with single-copy probes and the frequency of clones from mitochondrial or plastid DNA was estimated to be lower than 1 %.
Using the primers designed to amplify the 9_27 fragment, particularly forward primer 9_27-F and reverse primer 9_27-R as given in SEQ ID NO: 2 and SEQ ID NO: 3, respectively, the sugar beet BAC library can be screened by means of a PCR approach. The BAC library may also be screened by hybridisation to a polynucleotide as depicted in SEQ ID NO: 1 and SEQ ID NO: 4, respectively.
The BAC library developed from the commercial cultivar and calculated to represent 6 genome equivalents with an average insert size of 120 Kb (McGrath et al, 2004) is then screened for the presence of the polynucleotide according to the invention, particularly for the presence of the 9_27 fragment according to SEQ ID NO: 4. For the BACs identified in said screen end sequences (BES) are determined.
Further, primers may be designed to each of the individual BES sequences on each of the individual BAC clones identified in the primary screening and used in a second round of PCR based screening. As a result, additional BACs may be delivered which partially overlap with the existing BACs comprising the 9_27 fragment according to SEQ ID NO: 4 or parts thereof. The partially overlapping BACs can subsequently be 'tiled' based on the results of a PCR screening using primers designed to each of the individual BES sequences on each of the individual BAC clones, yielding a BAC contig around the 9_27 gene fragment
The nucleotide sequence of the selected BACs can then be analyzed by DNA sequencing and the so obtained nucleotide sequences assembled into one or more separate contigs. BLAST analysis of the contig sequences to the sugar beet EST collection supplemented by in silico gene analysis using gene prediction software allows for the identification of genes present on said contigs, including the gene comprising the 9_27 fragment.
Based on their homology to known flowering-time control genes or their putative regulatory function as suggested by the presence of conserved domains representative of regulatory proteins, few genes can be identified as potential candidates for the *B* gene. These genes need further validation by allelic variability and/or gene expression studies between annual and biennial genotypes, or by means of complementation or knockout experiments using transgenic approaches.
The B gene may be used in a transgenic approach for producing transgenic sugar beet plants comprising said polynucleotides stably integrated into the sugar beet genome. In particular, upon expression from the genome, the expression product can be used to modulate the vernalization response of the sugar beet plant.
In one aspect of the invention the vernalization response may be delayed by suppressing or down-regulating expression of the *B*-gene.
In another aspect of the invention, early bolting without cold treatment may be induced upon overexpression of the *B*-gene.

In the past molecular marker techniques have been developed which can be used for genetic mapping, gene cloning, marker assisted plant breeding and for genome fingerprinting and investigating genetic relationships. Genetic markers are based on DNA polymorphisms in the nucleotide sequences of genomic regions and can either be detected by restriction enzymes, or by means of two priming sites.
There are several types of molecular markers that may be used in marker-based selection including restriction fragment length polymorphism (RFLP), random amplification of polymorphic DNA (RAPD), amplified restriction fragment length polymorphism (AFLP), single sequence repeats (SSR) and single nucleotide polymorphisms SNPs.
The information content of the different types of markers may be different depending on the method that was used to obtain the marker data and the population in which the markers were scored. For example, it is not always possible to distinguish genome fragments that are present in homozygous condition from heterozygous fragments. In a heterogeneous population like an F₂, co-dominant markers like restriction fragment length polymorphisms (RFLPs, Botstein *et al.,* 1980) and co-dominantly scored amplified fragment length polymorphisms (AFLPs, Vos *et al.,* 1995) yield more information than dominant markers like random amplified polymorphic DNAs (RAPDs, Welsh and McCleland, 1990) and dominantly scored AFLPs. RFLPs are co-dominant and are able to identify a unique locus. RFLP involves the use of restriction enzymes to cut chromosomal DNA at specific short restriction sites, polymorphisms result from duplications or deletions between the sites or mutations at the restriction sites.
AFLP requires digestion of cellular DNA with a restriction enzyme before using PCR and selective nucleotides in the primers to amplify specific fragments. With this method up to 100 polymorphic loci can be measured and only relatively small DNA sample are required for each test.
The most preferred method of achieving such amplification of nucleotide fragments that span a polymorphic region of the plant genome employs the polymerase chain reaction ("PCR") (Mullis et al., Cold Spring Harbor Symp. Quant. Biol. 51:263 273 (1986) ;), using primer pairs involving a backward primer and a forward primer that are capable of hybridizing to the proximal sequences that define a polymorphism in its double-stranded form.
In contrast to RFLPs, PCR-based techniques require only a small percentage (approximately 10%) of the DNA amount as template to produce large quantities of the target sequence by PCR amplification.

One such PCR based technique is RAPD, which utilizes low stringency polymerase chain reaction (PCR) amplification with single primers of arbitrary sequence to generate strain-specific arrays of anonymous DNA fragments. The method requires only tiny DNA samples and analyses a large number of polymorphic loci. However, the unpredictable behaviour of short primers which is affected by numerous reaction conditions, inheritance in a dominant manner, and population specificity are the main disadvantages of RAPDs.
Microsatellites, or simple sequence repeats (SSRs), simple sequence length polymorphisms (SSLPs), short tandem repeats (STRs), simple sequence motifs (SSMs), and sequence target microsatellites (STMs) represent a class of repetitive sequences which are widely dispersed throughout the genome of eukaryotes. The variation in number and length of the repeats is a source of polymorphism even between closely related individuals. SSR analysis is based on these (short-repeat) sequences which are selectively amplified to detect variations in simple sequence repeats. Such microsatellite sequences can be easily amplified by PCR using a pair of flanking locus-specific oligonucleotides as primers and detect DNA length polymorphisms (Litt and Luty, 1989; Weber and May, 1989).
Mutations at a single nucleotide position resulting in substitutions, deletions or insertions give rise to single nucleotide polymorphisms or SNPs, which occur approximately every 1.3 kb in human (Cooper *et al.,* 1985; Kwok *et al.,* 1996). Most polymorphisms of this type have only two alleles and are also called biallelic loci. Positional cloning based on SNPs may accelerate the identification of disease traits and a range of biologically informative mutations (Wang *et al.,* 1998).
PCR extension assays that efficiently pick up point mutations may be used to detect SNPs. The procedure requires little DNA per sample. Three widely used types of SNP detection assays using PCR method are cleaved amplified polymorphic sequences (CAPS) (Konieczny and Ausubel, 1993; Thiel *et al.,* 2004), derived CAPS (dCAPS) (Michaels and Amasino, 1998; Neff *et al.,* 1998), and single strand conformation polymorphism (SSCP) (Orita *et al*., 1989).
CAPS polymorphisms are differences in restriction fragment lengths caused by SNPs or INDELs that create or abolish restriction endonuclease recognition sites in PCR amplicons produced by locus-specific oligonucleotide primers. CAPS assays are performed by digesting locus-specific PCR amplicons with one or more restriction enzymes and then separating the digested DNA on agarose or polyacrylamide gels.
dCAPS is a modification of the CAPS technique that allows detection of most single-nucleotide changes by utilizing mismatched PCR primers. Using the method, a restriction enzyme recognition site that includes the SNP is introduced into the PCR product by a primer containing one or more mismatches to template DNA. The PCR product modified in this manner is then subjected to restriction enzyme digestion, and the presence or absence of the SNP is determined by the resulting restriction pattern.
The SSCP technique separates denatured double stranded DNA on a non-denaturing gel, and thus allows the secondary structure, as well as the molecular weight, of single stranded DNA to determine gel mobility.
The ARMS (amplification refractory mutation system)-PCR procedure (Ye *et al.,* 2001) involves the use of a single PCR for SNP genotyping (Fan *et al.,* 2003; Chiapparino *et al.,* 2004). A tetra-primer, employing two primer pairs, is used to amplify two different alleles of a SNP in a single PCR reaction.
Alternative methods may be employed to amplify such fragments, such as the "Ligase Chain Reaction" ("LCR") (Barany, Proc. Natl. Acad. Sci. (U.S.A.) 88:189 193 (1991)), which uses two pairs of oligonucleotide probes to exponentially amplify a specific target. The sequences of each pair of oligonucleotides are selected to permit the pair to hybridize to abutting sequences of the same strand of the target. Such hybridization forms a substrate for a template-dependent ligase. As with PCR, the resulting products thus serve as a template in subsequent cycles and an exponential amplification of the desired sequence is obtained.

LCR can be performed with oligonucleotides having the proximal and distal sequences of the same strand of a polymorphic site. In one embodiment, either oligonucleotide will be designed to include the actual polymorphic site of the polymorphism. In such an embodiment, the reaction conditions are selected such that the oligonucleotides can be ligated together only if the target molecule either contains or lacks the specific nucleotide that is complementary to the polymorphic site present on the oligonucleotide. Alternatively, the oligonucleotides may be selected such that they do not include the polymorphic site (see, Segev, PCT Application WO 90/01069).

A further method that may alternatively be employed is the "Oligonucleotide Ligation Assay" ("OLA") (Landegren et al., Science 241:1077 1080 (1988)). The OLA protocol uses two oligonucleotides that are designed to be capable of hybridizing to abutting sequences of a single strand of a target. OLA, like LCR, is particularly suited for the detection of point mutations. Unlike LCR, however, OLA results in "linear" rather than exponential amplification of the target sequence.

Nickerson et al. have described a nucleic acid detection assay that combines attributes of PCR and OLA (Nickerson et al., Proc. Natl. Acad. Sci. (U.S.A.) 87:8923 8927 (1990)). In this method, PCR is used to achieve the exponential amplification of target DNA, which is then detected using OLA. In addition to requiring multiple, and separate, processing steps, one problem associated with such combinations is that they inherit all of the problems associated with PCR and OLA.

Schemes based on ligation of two (or more) oligonucleotides in the presence of a nucleic acid having the sequence of the resulting "di-oligonucleotide," thereby amplifying the di-oligonudeotide, are also known (Wu et al., Genomics 4:560 569 (1989)), and may be readily adapted to the purposes of the present invention.

Different assays based on the gene sequence according to the invention and as described herein above can thus be developed and used to screen plant material for the presence or absence of the annuality allele.
Molecular markers, preferentially End point TaqMan®, can be developed based on SNPs characterized from sequenced PCR products that are amplified from annual and biennial plants. Here, several PCR amplifications will be performed in order to cover the whole sequence of the gene.
New molecular markers will then be tested within different annual and biennial genetic backgrounds to evaluate the robustness of the molecular test.

In one embodiment, a molecular marker is a DNA fragment amplified by PCR, e.g. a SSR marker or a RAPD marker. In one embodiment, the presence or absence of an amplified DNA fragment is indicative of the presence or absence of the trait itself or of a particular allele of the trait. In one embodiment, a difference in the length of an amplified DNA fragment is indicative of the presence of a particular allele of a trait, and thus enables to distinguish between different alleles of a trait.

In a specific embodiment of the invention simple sequence repeat (SSR) markers are used to identify invention-relevant alleles in the parent plants and/or the ancestors thereof, as well as in the progeny plants resulting from a cross of said parent plants.
In another specific embodiment of the invention a marker based on a single nucleotide polymorphism is used to identify invention-relevant alleles in the parent plants and/or the ancestors thereof, as well as in the progeny plants resulting from a cross of said parent plants.
In still another embodiment of the invention a marker based on a deletion or an insertion ("INDEL") of at least one nucleotide is used to identify invention-relevant alleles in the parent plants and/or the ancestors thereof, as well as in the progeny plants resulting from a cross of said parent plants.
These markers can be developed based on the sequence of the polynucleotides according to the invention and as described herein before.

In particular, the markers according to the present invention can be used in an allelic discrimination assay, particularly in an assay for discriminating between different haplotypes within plant groupings of sugar beet plants exhibiting a biennial genotype. Said assay is based on a set of probe polynucleotides comprising two separate probe molecules that are complementary, for example, to a sub-region within an intronic region obtainable from the sugar beet genome by PCR amplification based on forward primer 9_27-F and reverse primer 9_27-R as given in SEQ ID NO: 2 and SEQ ID NO: 3, respectively, and differ only by one base mismatch, particularly a base mismatch at position #631; wherein a first probe, particularly a probe molecule which has a sequence as depicted in SEQ ID NO: 10 and is labelled with a first fluorescent dye such as, for example, FAM, more particularly with a first fluorescent dye and a quencher, represents one allele and a second probe, particularly a probe which has a sequence as depicted in SEQ ID NO: 11 and is labelled with a second fluorescent dye, which is not identical with the first dye, such as, for example VIC, more particularly with a second fluorescent dye and a quencher, represents the other allele.

The majority of commercial seed productions for sugar beet are done in southern France and northern Italy. In both regions, the presence of annual weed beets can cause pollen contamination in the seed productions, resulting in annuals in the commercial seed. This is not acceptable to a customer, and therefore all commercial seed lots are grown in regions, such as Argentina where no wild beets are growing directly after harvesting the seed. The plants are not vernalized and the presence of bolters is used to identify seed lots contaminated with annuals.

It is therefore an another aspect of the invention, to provide an assay involving markers that can discriminate specifically between annual plants and biennial plants and can thus be used, for example, for quality control of seed lots.
In particular, the invention relates to an assay, which is based on a set of probe polynucleotides comprising two separate probe molecules that are complementary, for example, to a sub-region within an intronic region obtainable from the sugar beet genome by PCR amplification based on forward primer 9_27-F and reverse primer 9_27-R as given in SEQ ID NO: 2 and SEQ ID NO: 3, respectively, and differ only by one base mismatch, particularly a base mismatch at position #598, wherein a first probe, particularly a probe molecule which has a sequence as depicted in SEQ ID NO: 14 and is labelled with a first fluorescent dye such as, for example, FAM, more particularly with a first fluorescent dye and a quencher, represents one allele and a second probe, particularly a probe which has a sequence as depicted in SEQ ID NO: 15 and is labelled with a second fluorescent dye, which is not identical with the first dye, such as, for example VIC, more particularly with a second fluorescent dye and a quencher, represents the other allele.
In another specific embodiment, the invention relates to an assay, which is based on a set of probe polynucleotides comprising two separate probe molecules that are complementary, for example, to a sub-region within a BES fragment of a BAC, which fragment can be obtained in a PCR reaction using a forward primer and reverse primer as given in SEQ ID NO:16 and SEQ ID NO: 17, respectively, and differ only by two base mismatches, particularly a base mismatch at positions #1179 and #1180, wherein a first probe, particularly a probe molecule which has a sequence as depicted in SEQ ID NO: 18 and is labelled with a first fluorescent dye such as, for example, FAM, more particularly with a first fluorescent dye and a quencher, represents one allele and a second probe, particularly a probe which has a sequence as depicted in SEQ ID NO: 19 and is labelled with a second fluorescent dye, which is not identical with the first dye, such as, for example VIC, more particularly with a second fluorescent dye and a quencher, represents the other allele.

### EXAMPLES

The following Examples provide illustrative embodiments. In light of the present disclosure and the general level of skill in the art, those of skill will appreciate that the following Examples are intended to be exemplary only and that numerous changes, modifications, and alterations can be employed without departing from the scope of the presently claimed subject matter.

### EXAMPLE 1: Characterization of the cDNA sequence fragment

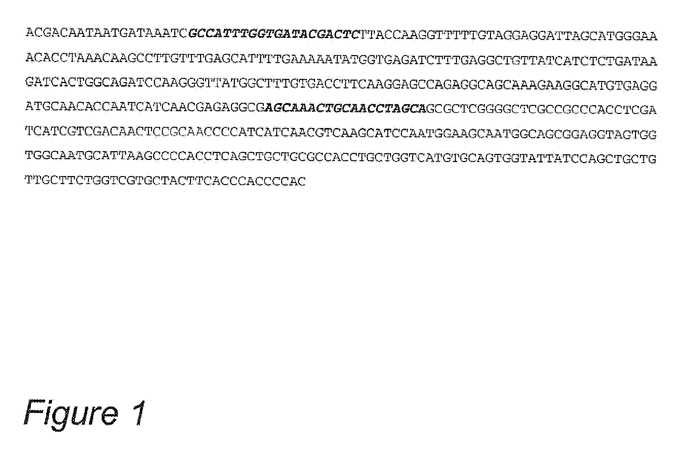

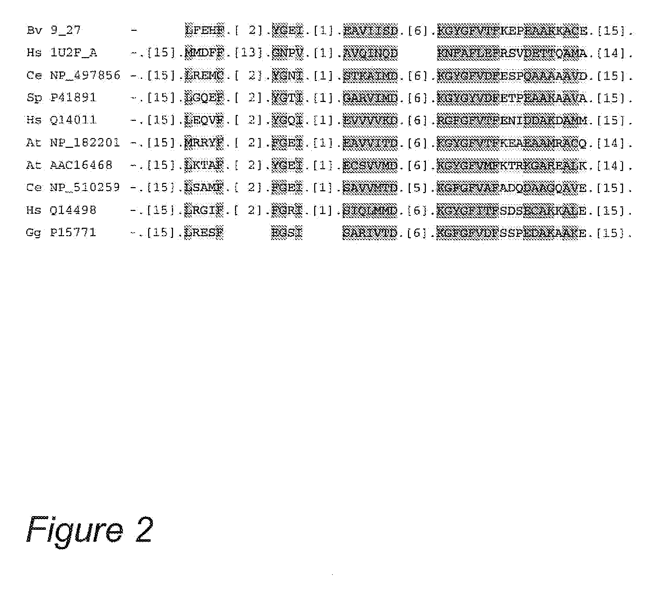

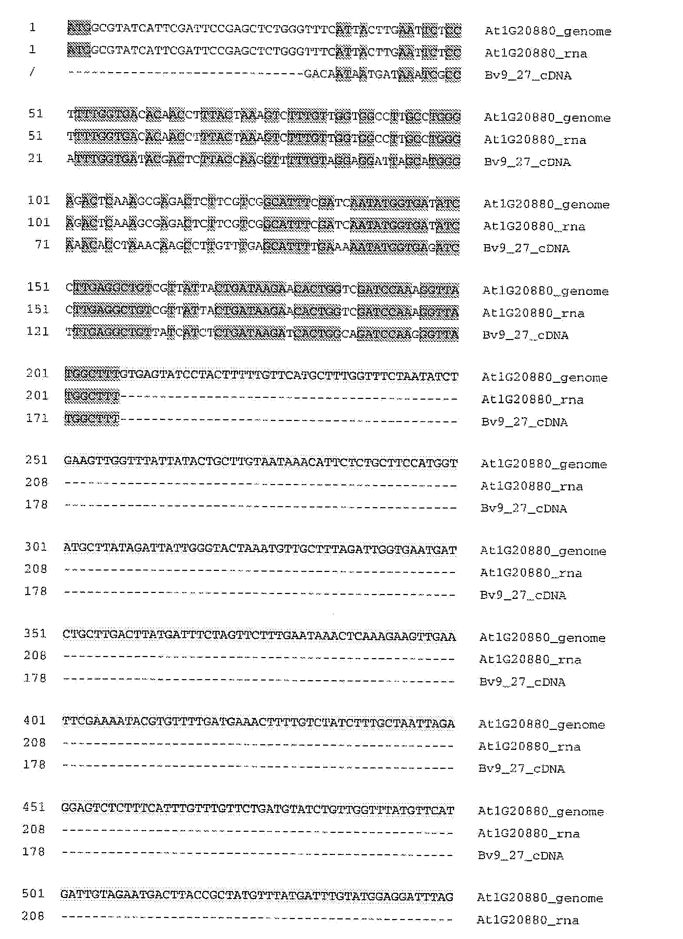

Figure 1 shows the nucleotide sequence of the 9_27 cDNA fragment. Homology searches in public sugar beet EST and GSS database using BLASTN returned no significant hits. Homology searches using the BLASTX function revealed the presence of a putative RNA recognition motif (RRM) in the corresponding amino acid sequence suggesting that the 9_27 gene belongs to a family of RNA binding proteins. Figure 2 shows the alignment of the RRM motif present at the 9_27 cDNA to different proteins from different species sharing the same RRM motif.

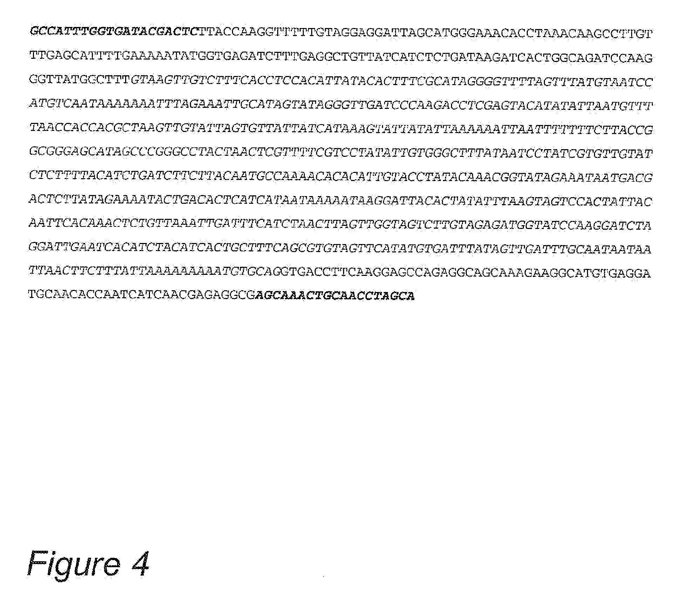

Based on the above observations, the putative gene structure was deduced using the alignment between the genomic sequence of the closest *Arabidopsis* homologue At1G20880 and the sugar beet 9_27 cDNA sequence that revealed the presence of a putative intronic region. A primer pair was designed encompassing the putative intronic region that subsequently delivered an amplification product of approximately 0.9 Kb using genomic DNA as template. Sequence analysis of the PCR product enabled the reconstruction of the genomic sequence of the 9_27 gene fragment, and confirmed the location of the intronic region of 610 base pairs in length (Figure 4).

### EXAMPLE 2: Allelic variability of the 9_27 fragment

Using the 9_27-F and 9_27-R primers described above, the genomic 9_27 fragment was amplified and sequenced across a panel of 1 annual and 16 biennial sugar beet lines. In total 3 different haplotypes were identified for the 16 biennial lines and one unique haplotype for the annual line. Based on these data, two assays were developed targeting the SNPs at position #598 and #631 (Figure 8) using the EndPoint TaqMan® technology from Applied Biosystems. Position #631 is polymorphic showing either an Adenine "A" or Cytosine "C" residue in different biennial lines. The annual line shows the most common Adenine "A" residue at position #631. Table 1 summarizes the nucleotide sequences of the primers and probes designed for the 9_27(T1) assay targeting SNP #631.

Table 2 lists all polymorphic positions discriminating the annual haplotype from all biennial haplotypes which includes the SNP at position #598. At this position, the annual haplotype shows a Guanine "G" instead of a Cytosine "C" residue present in all biennial haplotypes. The second assay, called 9_27(T2), that targets the SNP at position #598 thus potentially allows to differentiate the annual haplotype from all biennial haplotypes. Table 3 summarizes the primers and probes designed for assay 9_27(T2) targeting the SNP #598.

### EXAMPLE 3: Mapping of the 9_27 gene

Using the 9_27(T1) assay, the 9_27 gene was mapped at chromosome II in a F2S1 population of 192 individuals segregating for the SNP at position #631 and derived from a cross between two biennial lines (Figure 5). PCR amplification was performed using the TaqMan® Universal PCR Master Mix, No AmpErase® UNG (2X) from Applied Biosystems according to the manufacturer's recommendations. Cycling was performed as follows: 95°C for 10 min followed by 40 cycles of 95°C for 15 sec and 60°C for 1 min, using an ABI PRISM 7700 Sequence Detector instrument. EndPoint measurement was performed using the Sequence Detection System 2.0 software.
The 9_27 gene maps at a distance of 1 cM upstream of markers MP0176 and GJ01 and co-segregates with marker GJ131 (Möhring S. et al, 2004; Gaafar R. M. et al, 2005) (Figure 5). According to Gaafar et al. the *B* gene is predicted to be located in the interval delimited by markers GJ131 and GJ01.
Because of its position near the B gene, the 9_27 gene was subsequently mapped in a population segregating for the annuality trait. In this case, the 9_27(T2) assay was used to genotype a F2 population of 198 individuals from a cross between an annual and a biennial plant. PCR amplification was performed with 10 ng genomic DNA as template in a total volume of 10 µL, containing 0,25 mM dNTPs, 3 mM MgCl2, 0,2 µM of each primer, 0,1 µM of each probe, 0,3 µM of ROX and 0,5 U of ThermoWhite DNA polymerase (Saveen Werner AB). Cycling was performed as follows: 95°C for 2 min followed by 35 cycles of 95°C for 15 sec and 60°C for 1 min. The results of the 9_27(T2) assay show a perfect match with the phenotypic data and the predicted genotype of the *B* gene. Table 5 shows a fine-map of the B gene region for 9 individual progeny plants having the closest recombination events.

### EXAMPLE 4: BAC library screening and chromosome walking

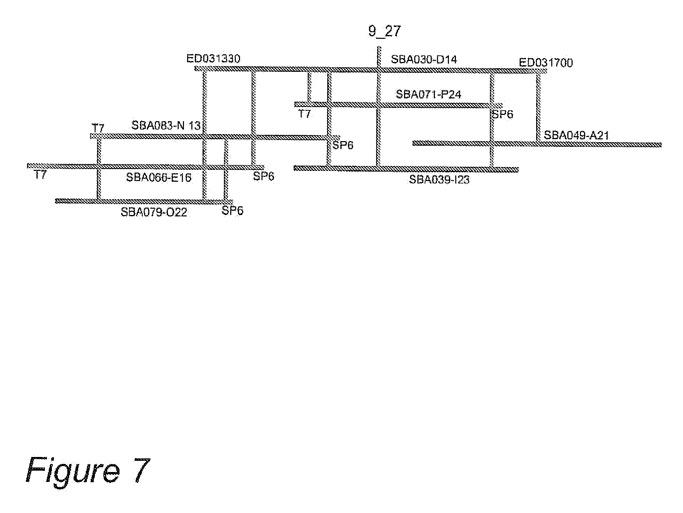

Using the primers 9_27-F and 9_27-R, a sugar beet BAC library was screened by means of PCR. The library was developed from the biennial commercial cultivar H20 and calculated to represent 6 genome equivalents with an average insert size of 120 Kb (McGrath et al, 2004). DNA pools for this library are commercialized by Amplicon Express, Pullman WA. Screening of the DNA pools for fragment 9_27 following the supplier's instructions resulted in the identification of BACs SBA030-D14, SBA071-P24 and SBA039-I23. For SBA030-D14 the BAC end sequences (BES) are publicly available under accession numbers ED031330 and ED031700. A second round of PCR screening using primers designed against both ED031330 and ED031700 delivered BACs SSA066-E16, SBA079-O22 and SBA083-N13 for ED031330, and SBA049-A21 for ED031700. The contig harboring fragment 9_27 thus comprises 7 partially overlapping BACs: SBA030-D14, SBA071-P24, SBA039-123, SBA066-E16, SBA079-O22, SBA083-N13 and SBA049-A21 that were all sent to MWG Biotech AG, Germany for BAC end sequencing. The BACs were subsequently 'tiled' based on the results of a PCR screening using primers designed to each of the individual BES sequences (Table 7) on each of the individual BAC clones, yielding a BAC contig around the 9_27 gene fragment (Fig. 7).
The PCR conditions for the screening of the DNA pools and the tiling of the BAC clones were as follows: primary denaturation at 95°C for 1 min followed by 40 amplification cycles of 30 seconds at 95°C, 30 seconds at 55°C and 90 seconds at 72°C. All PCR experiments were run at a GeneAMP PCR System 9700 instrument from Applied Biosystems Inc. using Platinum *Taq* DNA polymerase and the corresponding reaction mix from Invitrogen Corporation as recommended by the supplier.

### EXAMPLE 5: Fine mapping of the B gene

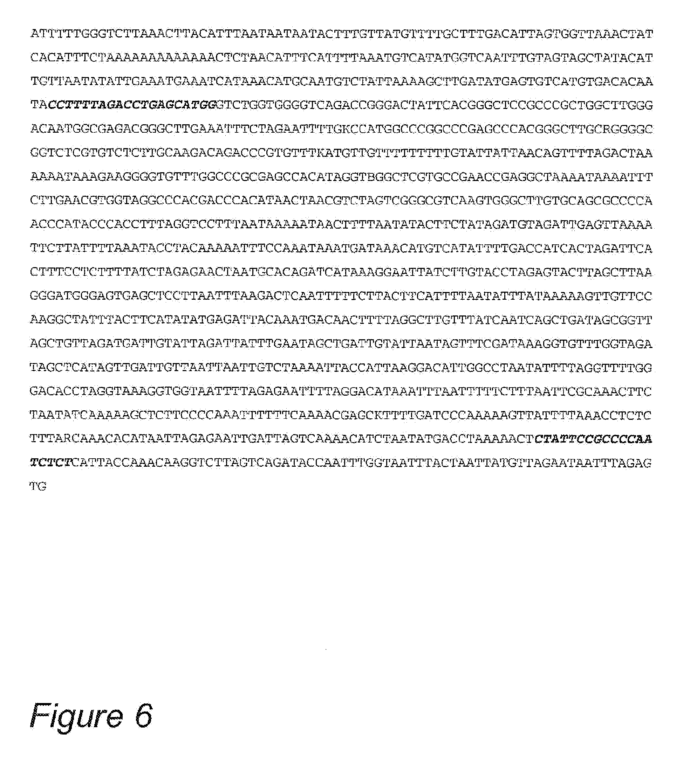

Sequence analysis of BES fragment ED031330 across the panel of 16 biennial lines used in EXAMPLE 2 revealed line 97151033 to have recombined between ED031330 and marker 9_27; 97151033 is biennial and accordingly carries a biennial genotype for 9_27, but the annual haplotype for ED031330. This observation limits the interval comprising the B gene to ED031330.
Sequence analysis of BES fragment ED031700 at the opposite extremity of BAC SBA030-D14 across the panel of the 16 biennial lines and the set of recombinant lines derived from the fine-mapping population for the B gene did not reveal any additional recombination events and therefore did not allow for the further delimitation of the interval. The nucleotide sequence of the PCR fragment obtained for ED031700 using primers ED031700-F and ED031700-R (Figure 6) revealed 5 different haplotypes: 4 haplotypes for the 16 biennial lines and one unique haplotype for the annual line. Table 5 lists all polymorphic positions discriminating the annual haplotype from all biennial haplotypes. At positions #1179 and #1180 the annual haplotype shows a T and a C residue instead of a double A residue in all biennial haplotypes. Based on these observations a Taqman® assay was developed targeting the SNPs at positions #1179 and #1180 (Table 6) that potentially allows to differentiate the annual haplotype from all biennial haplotypes. Assay ED031700(T1) was performed using the TaqMan® Universal PCR Master Mix, No AmpErase® UNG (2X) from Applied Biosystems according to the manufacturer's recommendations. PCR conditions were as follows: 95°C for 10 min followed by 40 cycles of 95°C for 15 sec and 60°C for 1 min, using an ABI PRISM 7700 Sequence Detector instrument. EndPoint measurement was performed using the Sequence Detection System 2.0 software.

### EXAMPLE 6: Correlation of the allelic variation of the 9_27 gene and the BES fragment ED031700 within annual and biennial genetic backgrounds:

Using the 9_27(T2) and ED031700(T1) molecular markers, a screening of a large panel of 243 different cultivated biennial sugar beet lines was performed. The results showed a perfect correlation between the biennial allelic form and the biennial phenotype of these cultivated sugar beet lines; none of the biennial lines carried the annual allelic form of both markers.
More detailed analysis of the allelic variation of the 9_27 and BES ED031700 fragments was undertaken by means of sequence analysis. None of the cultivated biennial lines was carrying the annual haplotypes characterized from the annual haplotypes identified in EXAMPLE 2 and 6. Additional biennial haplotypes were found, but the polymorphisms at position #598 for the 9_27 gene and at positions #1179 and #1180 for BES fragment ED031700, already described in the Table 2 and 5 respectively, revealed specific for the annual haplotype even across this larger panel.
Taken together, these data show that both the 9_27(T2) and the ED031700(T1) marker are perfectly correlated to the biennial growth habit across a large collection of 243 cultivated sugar beet lines. The strong linkage observed between both markers within the cultivated biennial genetic background probably is the result of the intense selection for this allele in sugar beet breeding starting from biennial ancestral plants.

### TABLES

**Table 1: Nucleotide sequences of primers and probes corresponding to assay 9_27(T1) for the genotyping of SNP #631**

| | |
|---|---|
| 9_27(T1)-F | TTGGTAGTSTTGTAGAGATGGTATCCA |
| 9_27(T1)-R | CCTCTGGCTCCTTGAAGGT |
| 9_27(T1)-FAM | FAM-ACTAGGATTGAATCACATC-MGB-NFQ |
| 9_27(T1)-VIC | VIC-TCTAGGATTGCATCACATC-MGB-NFQ |

**Table 2: Polymorphisms observed between annual and biennial sugar beet plants for the 9_27 gene fragment**

| **Position** | **BIENNIAL** | **ANNUAL** |
|---|---|---|
| 223 | C | T |
| 230 | A | G |
| 253 | C | G |
| 315 | C | T |
| 598 | C | G |
| 660 | G | A |
| 663 | G | . |
| 706 | C | G |
| 721 | A | . |

| | | |
|---|---|---|
| The first column indicates the nucleotidic position at the genomic sequence of the 9_27 fragment (Figure 4). The second and the third columns indicate the biennial and the annual haplotypes respectively. | | |

**Table 3: Nucleotide sequences of primers and probes corresponding to assay 9_27(T2) for the genotyping of SNP #598**

| precursor names | sequence (5' to 3') |
|---|---|
| 9_27(T2)-F | TGCCAAAACACACATTGTACCTATACA |
| 9_27(T2)-R | TGCCTCTGGCTCCTTGAAG |
| 9_27(T2)-FAM | FAM-ATCTCTACAAGACTACC-MGB-NFQ |
| 9_27(T2)-VIC | VIC-CATCTCTACAACACTACC-MGB-NFQ |

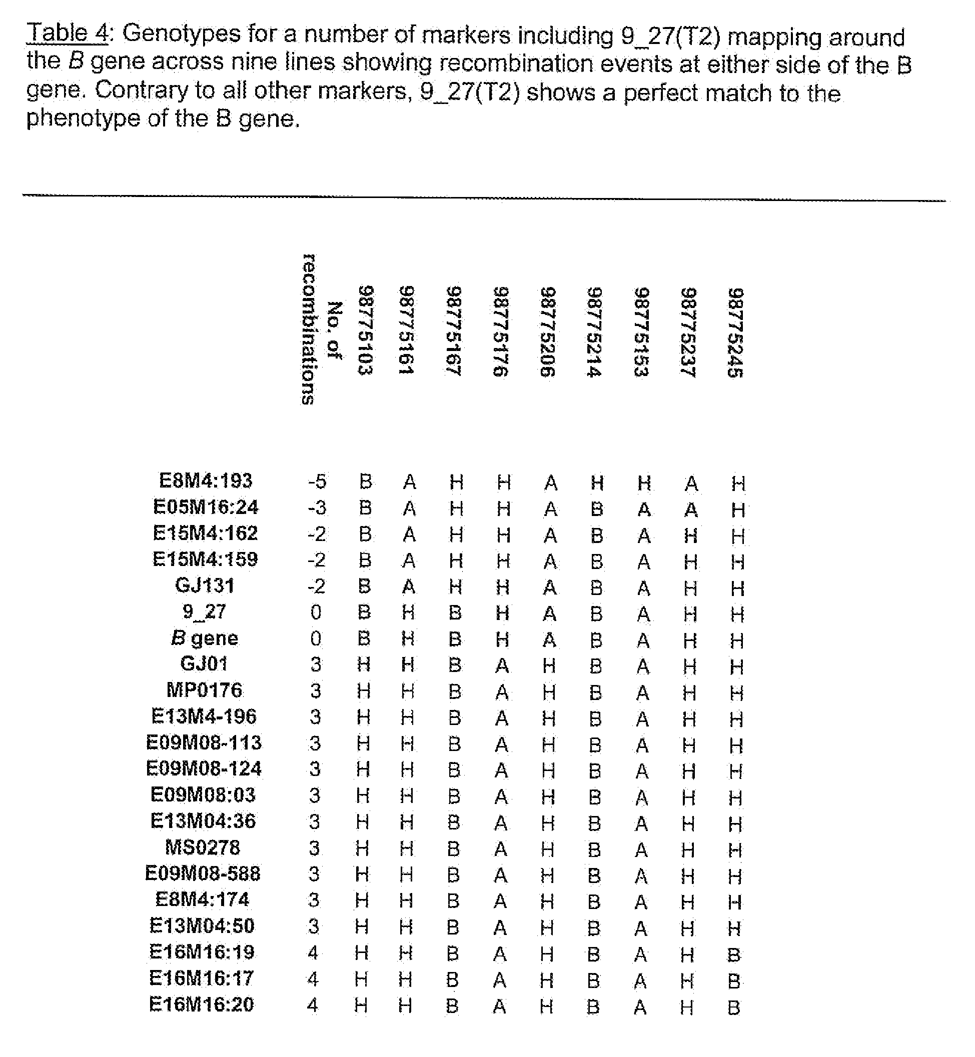

**Table 5: Polymorphisms observed between annual and biennial sugar beet plants for BES fragment ED031700**

| **Position** | **BIENNIAL** | **ANNUAL** |
|---|---|---|
| 1179 | A | T |
| 1180 | A | C |
| 1262 | A | T |

| | | |
|---|---|---|
| The first column indicates the nucleotidic position at the BES ED031700 genomic sequence (Figure 6). The second and the third columns indicate the biennial and the annual haplotypes respectively. | | |

**Table 6: Nucleotide sequences of primers and probes corresponding to assay ED031700 (T1) for the genotyping of SNPs #1179 and #1180.**

| precursor names | sequence (5' to 3') |
|---|---|
| ED031700(T1)-F | TAAAGGTGGTAATTTTAGAGAATTTTAGGA |
| ED031700(T1)-R | GCTCGTTTTGAAAAAATTTGGG |
| ED031700(T1)-FAM | FAM-TTAATTCGCAAACTTCT-MGB-NFQ |
| ED031700(T1)-VIC | VIC-TTTAATTCGCATCCTTCT-MGB-NFQ |

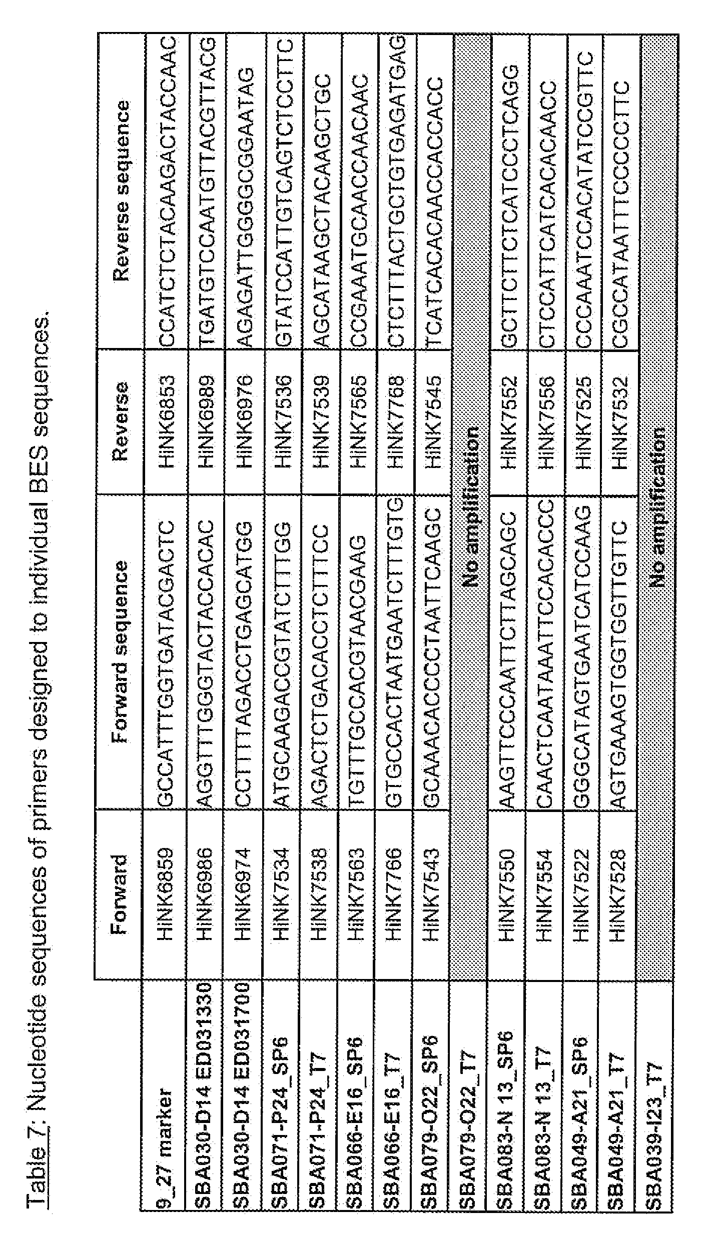

### References:

Abe *et al.,* 1997
Barany, Proc. Natl. Acad. Sci. (U.S.A.) 88:189 193 (1991)
Batzer, et al., Nucleic Acid Res. 19:5081 (1991)
Botstein *et al.,* 1980
Chiapparino *et al.* 2004
Cooper *et al.,* 1985
Fan *et al.,* 2003
Gaafar R. M., Hohmann U. and Jung C., 2005. Bacterial artificial chromosome-derived molecular markers for early bolting in sugar beet. TAG Theoretical and Applied Genetics, Volume 110, Number 6: 1027-1037
Konieczny and Ausubel, 1993
Kwok *et al.,* 1996
Landegren et al., Science 241:1077 1080 (1988)
Litt and Luty, 1989
McGrath J. M., Shaw R. S., de los Reyes B. G. and Weiland J. J., 2004. Construction of a Sugar Beet BAC Library from a Hybrid with diverse Traits. Plant Mol Biol Rep 22: 23-28
Michaels and Amasino, 1998
Möhring S., Salamini F. and Schneider K., 2005. Multiplexed, linkage group-specific SNP marker sets for rapid genetic mapping and fingerprinting of sugar beet (Beta vulgaris L.). Molecular Breeding, Volume 14, Number 4: 475-488
Mullis et al., Cold Spring Harbor Symp. Quant. Biol. 51:263 273 (1986)
Neff *et al.,* 1998
Nickerson et al., Proc. Natl. Acad. Sci. (U.S.A.) 87:8923 8927 (1990)
Ohtsuka, et al., J. Biol. Chem. 260:2605-2608 (1985)
Orita *et al.*, 1989
W.R. Pearson (1990), Methods in Enzymology 183, 63-98
Rossolini, et al., Mol. Cell. Probes 8:91-98 (1994)
Segev, PCT Application WO 90/01069
Smith and Waterman, Advances in Applied Mathematics 2 (1981), 482-489
Thiel *et al.,* 2004
Tijssen (1993) Laboratory Techniques in Biochemistry and Molecular Biology-Hybridization with Nucleic Acid Probes part I chapter 2 "Overview of principles of hybridization and the strategy of nucleic acid probe assays" Elsevier, New York.
Vos *et al.,* 1995
Wang *et al.,* 1998
Weber and May, 1989
Welsh and McCleland, 1990
Wu et al., Genomics 4:560 569 (1989)
Ye *et al.,* 2001

## Claims

1. A polynucleotide or an informative part or fragment thereof obtainable from a genomic sugar beet DNA genetically linked to the bolting gene or B gene comprising one or more of the following elements:
(a) an intronic region that can be obtained from the sugar beet genome by PCR amplification based on forward primer 9_27-F and reverse primer 9_27-R as given in SEQ ID NO: 2 and SEQ ID NO: 3, respectively;
(b) a polynucleotide fragment comprising a nucleotide sequence which has 70%, particularly 75%, more particularly 80%, even more particularly 85%, but especially 90% and up to 95%-99% sequence identity with a nucleotide sequence depicted in SEQ ID NO: 4;
(c) a polynucleotide fragment which, after splicing, has 70%, particularly 75%, more particularly 80%, even more particularly 85%, but especially 90% and up to 95%-99% sequence identity with a cDNA sequence shown in SEQ ID NO:1 and, optionally, in addition
(d) a nucleotide sequence encoding a RNA recognition motif domain (RRM).

2. A polynucleotide according to any of the preceding claims comprising a DNA sequence exhibiting a nucleotide sequence that hybridizes to the nucleotide sequence selected from the group of sequences depicted in SEQ ID NO: 1, SEQ ID NO: 4 and SEQ ID NO: 5, under high stringency conditions or to an informative fragment thereof.

3. A polynucleotide according to any of the preceding claims comprising a nucleotide sequence as shown in Figure 8 or an informative fragment thereof.

4. A polynucleotide according to claim 1 comprising a nucleotide sequences as shown in SEQ ID NO: 55 comprising an
a) A/C SNP at position #631; or
b) G/C SNP at position #598; or
c) A/T SNP at position #1179 and an A/C SNP at position 1180.

5. A polynucleotide or an informative part or fragment thereof according to any of the preceding claims which shows perfect co-segregation with the bolting gene (*B*-gene) associated phenotype in sugar beet.

6. A polynucleotide or an informative part or fragment thereof obtainable from at least one BAC selected from a first group of partially overlapping BACs which BACs can be identified and isolated from a BAC library developed from the biennial commercial cultivar H20 and tiled based on their BAC end sequences (BES) which can be amplified using a primer pair selected from the group consisting of:
- primer pair 2 represented by a forward primer of SEQ ID NO: 34 and a reverse primer of SEQ ID NO: 35,
- primer pair 3 represented by a forward primer of SEQ ID NO: 21 and a reverse primer of SEQ ID NO: 22,
- primer pair 4 represented by a forward primer of SEQ ID NO: 36 and a reverse primer of SEQ ID NO: 37,
- primer pair 5 represented by a forward primer of SEQ ID NO: 38and a reverse primer of SEQ ID NO: 39,
- primer pair 6 represented by a forward primer of SEQ ID NO: 40 and a reverse primer of SEQ ID NO: 41,
- primer pair 7 represented by a forward primer of SEQ ID NO: 42 and a reverse primer of SEQ ID NO: 43,
- primer pair 8 represented by a forward primer of SEQ ID NO: 44 and a reverse primer of SEQ ID NO: 45
- primer pair 9 represented by a forward primer of SEQ ID NO: 46 and a reverse primer of SEQ ID NO: 47,
- primer pair 10 represented by a forward primer of SEQ ID NO: 48 and a reverse primer of SEQ ID NO: 49,
- primer pair 11 represented by a forward primer of SEQ ID NO: 50 and a reverse primer of SEQ ID NO: 51,
- primer pair 12 represented by a forward primer of SEQ ID NO: 52 and a reverse primer of SEQ ID NO: 53.

7. A polynucleotide according to claim 6 or an informative part or fragment thereof, which polynucleotide is **characterized by** the end sequence selected from the group of sequences shown in SEQ ID NO: 20; SEQ ID NO: 28; SEQ ID NO: 29; SEQ ID NO: 23; SEQ ID NO: 26; SEQ ID NO: 27; SEQ ID NO: 30; SEQ ID NO: 31; SEQ ID NO: 32; SEQ ID NO: 33; SEQ ID NO: 24; SEQ ID NO: 25.

8. A pair of primers consisting of a forward primer and a reverse primer which primers are capable of amplifying a fragment of the polynucleotide according to any of the preceding claims or of an informative part thereof, wherein said fragment comprises a polymorphism, particularly a polymorphism selected from the group consisting of an SNP, an SSR, a deletion or an insertion of at least one nucleotide, which polymorphism is diagnostic for the B allele at the *B* locus and allows to discriminate between the annual and biennial genotype.

9. A pair of primers according to claim 8, wherein the polymorphism is based on an SNP selected from the group of SNP #598; SNP #631; and SNP #1179/1180.

10. Use of a polynucleotide or of a pair or primers according to any of the preceding claims or any informative fragment thereof as a marker in an allelic discrimination assay for detecting a polymorphism in the sugar beet genome.

11. A method of identifying the absence or presence of an allele associated with annuality in a sugar beet plant comprising
a) obtaining a genomic sample from a sugar beet plant to be analyzed,
b) analyzing the nucleotide sequence of a genomic region from said sample complementary to the sequence of a polynucleotide according to any of the preceeding claims using a molecular marker based on a polynucleotide or an informative fragment thereof or a pair of primers according to any of the preceding claims, and
c) comparing said sequence with an allelic sequence known to be associated with the biennial phenotype and the annual phenotype, respectively.

12. An allelic discrimination assay for detecting a polymorphism in a genomic region of the sugar beet genome co-segregating with the annuality phenotype comprising a molecular marker based on a polynucleotide according to any of the preceding claims or any informative fragment thereof.

13. An allelic discrimination assay according to claim 68, wherein said molecular marker comprises a pair of primers according to any of the preceding claims.

14. Use of a polynucleotide according to any of the preceding claims for the development of a molecular marker to be used for identifying the absence or presence of an allele associated with annuality in a sugar beet genome comprising
a) identifying in said polynucleotide polymorphic sites
b) associating said polymorphisms with the absence or presence of an allele associated with annuality in sugar beet by
c) designing forward and reverse primers around this polymorphic site that can be used in an allelic discrimination assay.
